(19) Europäisches Patentamt
European Patent Office
Office européen des brevets

(11) EP 3 039 156 B1

(12) # EUROPEAN PATENT SPECIFICATION

(45) Date of publication and mention of the grant of the patent:
**02.01.2019 Bulletin 2019/01**

(21) Application number: **14758368.6**

(22) Date of filing: **29.08.2014**

(51) Int Cl.:
***C12Q 1/6883*** *(2018.01)*

(86) International application number:
**PCT/EP2014/068387**

(87) International publication number:
**WO 2015/028612 (05.03.2015 Gazette 2015/09)**

(54) **RISK MARKERS FOR CARDIOVASCULAR DISEASE IN PATIENTS WITH CHRONIC KIDNEY DISEASE**

RISIKOMARKER FÜR KARDIOVASKULÄRE ERKRANKUNG BEI PATIENTEN MIT CHRONISCHER NIERENERKRANKUNG

MARQUEURS DE RISQUE DE MALADIE CARDIOVASCULAIRE CHEZ DES PATIENTS ATTEINTS DE MALADIE RÉNALE CHRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.08.2013 EP 13182484**

(43) Date of publication of application:
**06.07.2016 Bulletin 2016/27**

(73) Proprietor: **Gendiag.exe, S.L.**
**08950 Esplugues de Llobregat (Barcelona) (ES)**

(72) Inventors:
- **SALAS, Eduardo**
  **08950 Esplugues de Llobregat (Barcelona) (ES)**
- **PICH, Sara**
  **08950 Esplugues de Llobregat (Barcelona) (ES)**
- **ARIAS, Manuel**
  **08950 Esplugues de Llobregat (Barcelona) (ES)**
- **ELOSUA, Roberto**
  **08950 Esplugues de Llobregat (Barcelona) (ES)**
- **CASTILLO, Sergio**
  **08950 Esplugues de Llobregat (Barcelona) (ES)**

(74) Representative: **Hoffmann Eitle**
**Patent- und Rechtsanwälte PartmbB**
**Arabellastraße 30**
**81925 München (DE)**

(56) References cited:
**EP-A1- 2 264 183** **EP-A1- 2 554 679**
**WO-A1-2012/001613** **WO-A2-2008/060618**

- **PATRICK LINSEL-NITSCHKE ET AL: "Genetic variation in the arachidonate 5-lipoxygenase-activating protein (ALOX5AP) is associated with myocardial infarction in the German population", CLINICAL SCIENCE, vol. 115, no. 10, 1 November 2008 (2008-11-01), page 309, XP055013105, ISSN: 0143-5221, DOI: 10.1042/CS20070468**
- **ANNABEL Z. WANG ET AL: "Association of SNP rs17465637 on Chromosome 1q41 and rs599839 on 1p13.3 with Myocardial Infarction in an American Caucasian Population", ANNALS OF HUMAN GENETICS, vol. 75, no. 4, 4 April 2011 (2011-04-04), pages 475-482, XP055013117, ISSN: 0003-4800, DOI: 10.1111/j.1469-1809.2011.00646.x**
- **YAN YU ET AL: "Evaluation of population impact of candidate polymorphisms for coronary heart disease in the Framingham Heart Study Offspring Cohort", BMC PROCEEDINGS, BIOMED CENTRAL LTD, LONDON UK, vol. 3, no. Suppl 7, 15 December 2009 (2009-12-15), page S118, XP021069893, ISSN: 1753-6561**
- **YUMIKO HIURA ET AL: "Validation of the Association of Genetic Variants on Chromosome 9p21 and 1q41 With Myocardial Infarction in a Japanese Population", CIRCULATION JOURNAL, vol. 72, no. 8, 1 August 2008 (2008-08-01) , pages 1213-1217, XP055013121, ISSN: 1346-9843, DOI: 10.1253/circj.72.1213**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 039 156 B1

- EAGLE K A ET AL: "Summaries of Key Journal Articles", JOURNAL OF THE AMERICAN COLLEGE OF CARDIOLOGY, ELSEVIER, NEW YORK, NY, US, vol. 55, no. 9, 2 March 2010 (2010-03-02), pages 948-955, XP026921139, ISSN: 0735-1097, DOI: 10.1016/J.JACC.2010.02.001 [retrieved on 2010-02-23]
- M RAO ET AL: "Cytokine gene polymorphism and progression of renal and cardiovascular diseases", KIDNEY INTERNATIONAL, vol. 72, no. 5, 20 June 2007 (2007-06-20), pages 549-556, XP055087743, ISSN: 0085-2538, DOI: 10.1038/sj.ki.5002391
- ARKING D E ET AL: "Understanding cardiovascular disease through the lens of genome-wide association studies", TRENDS IN GENETICS, ELSEVIER SCIENCE PUBLISHERS B.V. AMSTERDAM, NL, vol. 25, no. 9, 1 September 2009 (2009-09-01), pages 387-394, XP026564456, ISSN: 0168-9525, DOI: 10.1016/J.TIG.2009.07.007 [retrieved on 2009-08-26]
- ROBERT CLARKE ET AL: "Genetic Variants Associated with Lp(a) Lipoprotein Level and Coronary Disease", NEW ENGLAND JOURNAL OF MEDICINE, vol. 361, no. 26, 24 December 2009 (2009-12-24), pages 2518-2528, XP055023613, ISSN: 0028-4793, DOI: 10.1056/NEJMoa0902604

SEQUENCE LISTING

<110> Gendiag.exe, S.L.

<120> RISK MARKERS FOR CARDIOVASCULAR DISEASE IN PATIENTS WITH CHRONIC KIDNEY DISEASE

<130> HE 167 141

<160> 69

<170> PatentIn version 3.5

<210> 1
<211> 53
<212> DNA
<213> Homo sapiens

<400> 1
aaccataata gttatgctga gaagttcttt tttgtcatag tgcaagataa cat 53

<210> 2
<211> 52
<212> DNA
<213> Homo sapiens

<400> 2
gctatcattt aaatttggtt gagacacaat atgctgttgc actttctata aa 52

<210> 3
<211> 52
<212> DNA
<213> Homo sapiens

<400> 3
ctgtgctgct tggtgcctct ctgatatgaa tacactgaca cgtcaaagta ac 52

<210> 4
<211> 52
<212> DNA
<213> Homo sapiens

<400> 4
acatctgcct ctctagacta taaactcttt ggggctaggt cttctttgtc tt 52

<210> 5
<211> 52
<212> DNA
<213> Homo sapiens

<400> 5
tcatactaac catatgatca acagttcaaa agcagccact cgcagaggta ag 52

<210> 6
<211> 52
<212> DNA
<213> Homo sapiens

<400> 6
ttgaaaaaaa ttaattctca cactccaaag tgcatttaat ttaagctact tt 52

<210> 7
<211> 52
<212> DNA
<213> Homo sapiens

<400> 7
ggcaagtacc tgggcacagg gctgcttcat ggccttggac ctggacagtg ga 52

<210> 8
<211> 53
<212> DNA
<213> Homo sapiens

<400> 8
ttcagacacc ttgttctcag aacccagtgt gtttatacag gttagaggag aaa 53

<210> 9
<211> 52

<212> DNA
<213> Homo sapiens

<400> 9
tgtccaagcc tctctttgca attctaatta acctcaatgt tgcaaccata ga 52

<210> 10
<211> 52
<212> DNA
<213> Homo sapiens

<400> 10
ctcatgaaca tgactgtgaa caggaaaaca gggagagaat gaagctggcc aa 52

<210> 11
<211> 52
<212> DNA
<213> Homo sapiens

<400> 11
gagttttcct gggatgtggt cctttcggtt ttttaaaaaat tatttttatt ga 52

<210> 12
<211> 21
<212> DNA
<213> Homo sapiens

<400> 12
atcaacagtt gaaaagcagc c 21

<210> 13
<211> 21
<212> DNA
<213> Homo sapiens

<400> 13
gtcactaccc tactgtcatt c 21

<210> 14
<211> 21
<212> DNA
<213> Homo sapiens

<400> 14
atcaacagtt caaaagcagc c 21

<210> 15
<211> 21
<212> DNA
<213> Homo sapiens

<400> 15
aatgtgactg cttctgcata c 21

<210> 16
<211> 21
<212> DNA
<213> Homo sapiens

<400> 16
acacattggg ttctgagaac a 21

<210> 17
<211> 20
<212> DNA
<213> Homo sapiens

<400> 17
acttcctgac tctcagctgc 20

<210> 18
<211> 20
<212> DNA
<213> Homo sapiens

<400> 18
acacactggg ttctgagaac 20

<210> 19
<211> 23
<212> DNA
<213> Homo sapiens

<400> 19
aagtgaagga tatctatatt gac 23

<210> 20
<211> 22
<212> DNA
<213> Homo sapiens

<400> 20
aacagcatat tatgtctcaa cc 22

<210> 21
<211> 21
<212> DNA
<213> Homo sapiens

<400> 21
aaaggaatta tgggtgagca g 21

<210> 22
<211> 22
<212> DNA
<213> Homo sapiens

<400> 22
aacagcatat tgtgtctcaa cc 22

<210> 23
<211> 22
<212> DNA
<213> Homo sapiens

<400> 23
ttggcagatg ctttatagaa ag 22

<210> 24
<211> 21
<212> DNA
<213> Homo sapiens

<400> 24
ctctctgata cgaatacact g 21

<210> 25
<211> 20
<212> DNA
<213> Homo sapiens

<400> 25
tcagatctgt ctcttgctgc 20

<210> 26
<211> 22
<212> DNA
<213> Homo sapiens

<400> 26
ctctctgata tgaatacact ga 22

<210> 27
<211> 21
<212> DNA
<213> Homo sapiens

<400> 27
tgaactggag ttttctggat g 21

<210> 28
<211> 24
<212> DNA
<213> Homo sapiens

<400> 28
ttgcaattct atttaacctc aatg 24

<210> 29
<211> 21
<212> DNA
<213> Homo sapiens

<400> 29
agatccagat gtatgtccaa g 21

<210> 30
<211> 24
<212> DNA
<213> Homo sapiens

<400> 30
attctaatta acctcaatgt tgca 24

<210> 31
<211> 21

<212> DNA
<213> Homo sapiens

<400> 31
actctgtaag gtaggtctat g 21

<210> 32
<211> 18
<212> DNA
<213> Homo sapiens

<400> 32
agggctgctc catggcct 18

<210> 33
<211> 21
<212> DNA
<213> Homo sapiens

<400> 33
aatctgaagg tttagggcaa g 21

<210> 34
<211> 19
<212> DNA
<213> Homo sapiens

<400> 34
agggctgctt catggcctt 19

<210> 35
<211> 20
<212> DNA
<213> Homo sapiens

<400> 35
ttcaagatgg cagcagcaga 20

<210> 36
<211> 20
<212> DNA
<213> Homo sapiens

<400> 36
tgaacaggaa aacagggaga 20

<210> 37
<211> 21
<212> DNA
<213> Homo sapiens

<400> 37
agctagttag gatgtcttat c 21

<210> 38
<211> 19
<212> DNA
<213> Homo sapiens

<400> 38
tgaacaggaa gacagggag 19

<210> 39
<211> 19
<212> DNA
<213> Homo sapiens

<400> 39
aagcactgct agctgacac 19

<210> 40
<211> 21
<212> DNA
<213> Homo sapiens

<400> 40
actataaact ctttggggct a 21

<210> 41
<211> 20
<212> DNA
<213> Homo sapiens

<400> 41
gtatggtgac ccagtacttc 20

<210> 42
<211> 21
<212> DNA
<213> Homo sapiens

<400> 42
actataaact gtttggggct a 21

<210> 43
<211> 21
<212> DNA
<213> Homo sapiens

<400> 43
atgcaagaaa gtgtgctaga c 21

<210> 44
<211> 23
<212> DNA
<213> Homo sapiens

<400> 44
tgtggtcctt tcggtttttt aaa 23

<210> 45
<211> 20
<212> DNA
<213> Homo sapiens

<400> 45
ctgattggcc tggcattgag 20

<210> 46
<211> 24
<212> DNA
<213> Homo sapiens

<400> 46
tgtggtcctt tcagtttttt aaaa 24

<210> 47
<211> 23
<212> DNA
<213> Homo sapiens

<400> 47
aatgttctca acacatagaa atg 23

<210> 48
<211> 53
<212> DNA
<213> Homo sapiens

<400> 48
agaagatgtg gaggaaatgg aagtatctat acactgctga tggggatgtg aag 53

<210> 49
<211> 53
<212> DNA
<213> Homo sapiens

<400> 49
gaagggtaaa gggtggtagg attgagctga gtcaggccag aaacctctag tta 53

<210> 50
<211> 46
<212> DNA
<213> Homo sapiens

<400> 50
acttcgtcag taacggacca tgttatcttg cactatgaca aaaaat 46

<210> 51
<211> 46
<212> DNA
<213> Homo sapiens

<400> 51
gagtcgaggt catatcgtca tgttatcttg cactatgaca aaaaag 46

<210> 52
<211> 65
<212> DNA
<213> Homo sapiens

<400> 52

acttctcagc ataactatta tggttgcaac gatcctacag tatccgcgtc tgcctatagt 60

gagtc 65

<210> 53
<211> 22
<212> DNA
<213> Homo sapiens

<400> 53
gcaacgatcc tacagtatcc gc 22

<210> 54
<211> 45
<212> DNA
<213> Homo sapiens

<400> 54
acttcgtcag taacggactt gaaaaaaatt aattctcaca ctcca 45

<210> 55
<211> 45
<212> DNA
<213> Homo sapiens

<400> 55
gagtcgaggt catatcgttt gaaaaaaatt aattctcaca ctccg 45

<210> 56
<211> 64
<212> DNA
<213> Homo sapiens

<400> 56

agtgcattta atttaagcta cttttctccg gctgatggaa ccgtaggtct gcctatagtg 60

agtc 64

<210> 57
<211> 22
<212> DNA
<213> Homo sapiens

<400> 57
tctccggctg atggaaccgt ag 22

<210> 58
<211> 22
<212> DNA
<213> Homo sapiens

<400> 58
ctgattttac catgtgttgg ag 22

<210> 59

<211> 22
<212> DNA
<213> Homo sapiens

<400> 59
ttcaagatag ctgtaccact tc 22

<210> 60
<211> 27
<212> DNA
<213> Homo sapiens

<400> 60
aatggaagta tcatacactg ctgatgg 27

<210> 61
<211> 28
<212> DNA
<213> Homo sapiens

<400> 61
aaatggaagt attatacact gctgatgg 28

<210> 62
<211> 20
<212> DNA
<213> Homo sapiens

<400> 62
aggactgaac agagactgag 20

<210> 63
<211> 21
<212> DNA
<213> Homo sapiens

<400> 63
gttgaatgca tgtttccctt c 21

<210> 64
<211> 19
<212> DNA
<213> Homo sapiens

<400> 64
aggattgagc gagtcaggc 19

<210> 65
<211> 20
<212> DNA
<213> Homo sapiens

<400> 65
taggattgag tgagtcaggc 20

<210> 66
<211> 43
<212> DNA

<213> Homo sapiens

<400> 66
acttcgtcag taacggacgt cacatcccca tcagcagtgt ata 43

<210> 67
<211> 43
<212> DNA
<213> Homo sapiens

<400> 67
gagtcgaggt catatcgtgt cacatcccca tcagcagtgt atg 43

<210> 68
<211> 62
<212> DNA
<213> Homo sapiens

<400> 68

**tacttccatt tcctccacat ctcctgatac gcgagcctag acgtgtctgc ctatagtgag 60**

**tc 62**

<210> 69
<211> 22
<212> DNA
<213> Homo sapiens

<400> 69
cctgatacgc gagcctagac gt 22

## Description

## FIELD OF THE INVENTION

The present invention relates to the field of cardiovascular diseases or disorders. More specifically, it relates to markers and methods for determining whether a subject, particularly a human subject suffering from chronic kidney disease, is at risk of developing cardiovascular disease, having cardiovascular disease, or experiencing a complication of a cardiovascular disease. The present invention also relates to the use of such markers and methods for monitoring the status of cardiovascular risk and/or disease in a subject suffering from chronic kidney disease or the effects of preventive and/or therapeutic measures/agents on subjects suffering from chronic kidney disease with cardiovascular risk or cardiovascular disease.

## TECHNICAL BACKGROUND

### 1.- Chronic Kidney Disease

Chronic kidney disease is a worldwide public health problem. There is a rising incidence and prevalence of kidney failure, with poor outcome and high cost. There is an even higher prevalence of earlier stages of chronic kidney disease (Am J Kidney Dis 39:S1-S266, 2002 suppl 1).

Increasing evidence, accrued in the past decades, indicates that the adverse outcome of chronic kidney disease, such as kidney failure, cardiovascular disease, and premature death, can be prevented or delayed. Earlier stages of chronic kidney disease can be detected through laboratory testing. Treatment of earlier stages of chronic kidney disease is effective in slowing the progression toward kidney failure. Initiation of treatment for cardiovascular risk factors at earlier stages of chronic kidney disease should be effective in reducing cardiovascular disease events both before and after the onset of kidney failure (Am J Kidney Dis 39:S1-S266, 2002 suppl 1).

Unfortunately, chronic kidney disease is "under-diagnosed" and "under-treated", resulting in insufficient prevention of cardiovascular disease (Am J Kidney Dis 39:S1-S266, 2002 suppl 1, J Am Coll Cardiol 2007; 50: 217).

The criteria for the definition of chronic kidney disease (CKD) are: Kidney damage for ≥3 months, as defined by structural or functional abnormalities of the kidney, with or without decreased Glomerular Filtration Rate (GFR), that can lead to decreased GFR, manifest by either pathological abnormalities or markers of kidney damage, including abnormalities in imaging test or a GFR <60 mL/min/11,73m$^2$ for ≥ 3 months, with or without kidney damage (Am J Kidney Dis 39:S1-S266, 2002 suppl 1).

Five stages of CKD have been defined (Am J Kidney Dis 39:S1-S266, 2002 suppl 1):

| | | Classification by severity | |
|---|---|---|---|
| Stage | Description | GFR mL/min/1.73 m$^2$ | Related terms |
| 1 | Kidney damage with normal or ↑ GFR | ≥ 90 | Albuminuria, proteinuria, hematuria |
| 2 | Kidney damage with mild ↓ GFR | 60-89 | Albuminuria, proteinuria, hematuria |
| 3 | Moderate ↓ GFR | 30-59 | Chronic renal insufficiency, early renal insufficiency |
| 4 | Severe ↓ GFR | 15-29 | Chronic renal insufficiency, late renal insufficiency, pre-ESRD |
| 5 | Kidney failure | < 15 (or dialysis) | Renal failure, uremia, end-stage renal disease |

The prevalence of each stage of CKD has also been established (Am J Kidney Dis 32:992-999, 1998, erratum 35:178, 2000, US Renal Data System. USRDS 1998 Annual Data Report, National Institutes of Health, National Institute of Diabetes and Digestive and Kidney Diseases, Bethesda, MD, 1998):

| Stage | Prevalence (Age ≥ 20) |
|---|---|
| 1 | 3.3 % |
| 2 | 3.0 % |
| 3 | 4.3 % |
| 4 | 0.2 % |
| 5 | 0.1 % |

**2.- Treatment of CKD** The treatment of CKD also generally follows several stages, described comprehensively in Harrison's Principles of Internal Medicine. 18th ed. New York: McGraw-Hill; 2012):

| Stage | Description | GFR mL/min per 1.73 $m^2$ | Action* |
|---|---|---|---|
| 1 | Kidney damage with normal or ↑ GFR | ≥9 0 | Diagnosis and treatment, treatment of comorbid conditions, slowing progression, CVD risk reduction |
| 2 | Kidney damage with mild ↓ GFR | 60-89 | Estimating progression |
| 3 | Moderate ↓ GFR | 30-59 | Evaluating and treating complications |
| 4 | Severe ↓ GFR | 15-29 | Preparation for kidney replacement therapy |
| 5 | Kidney failure | <15 (or dialysis) | kidney replacement (if uremia present) |

Treatments aimed at specific causes of CKD include optimized glucose control in diabetes mellitus, immunomodulatory agents for glomerulonephritis, and emerging specific therapies to retard cytogenesis in polycystic kidney disease. The optimal timing of both specific and nonspecific therapy is usually well before there has been a measurable decline in GFR and certainly before CKD is established. It is helpful to sequentially measure and plot the rate of decline of GFR in all patients. Any acceleration in the rate of decline should prompt a search for superimposed acute or subacute processes that may be reversible. These include Extra Cellular Fluid Volume (ECFV) depletion, uncontrolled hypertension, urinary tract infection, new obstructive uropathy, exposure to nephrotoxic agents [such as nonsteroidal anti-inflammatory drugs (NSAIDs) or radiographic dye], and reactivation or flare of the original disease, such as lupus or vasculitis.

The following interventions should be considered in an effort to stabilize or slow the decline of renal function:

- Reducing Intraglomerular Hypertension and Proteinuria
- Slowing Progression of Diabetic Renal Disease
    - Control of Blood Glucose
    - Control of Blood Pressure and Proteinuria
- Protein Restriction
- Managing Other Complications of Chronic Kidney Disease
- Medication Dose Adjustment
- Preparation for Renal Replacement Therapy

Temporary relief of symptoms and signs of impending uremia, such as anorexia, nausea, vomiting, lassitude, and pruritus, may sometimes be achieved with protein restriction. However, this carries a significant risk of protein-energy malnutrition, and thus plans for more long-term management should be in place.

Maintenance dialysis and kidney transplantation has extended the lives of hundreds of thousands of patients with CKD worldwide. Clear indications for initiation of renal replacement therapy for patients with CKD include uremic pericarditis, encephalopathy, intractable muscle cramping, anorexia, and nausea not attributable to reversible causes such as peptic ulcer disease, evidence of malnutrition, and fluid and electrolyte abnormalities, principally hyperkalemia or ECF volume overload, that are refractory to other measures.

Recommendations for the optimal time for initiation of renal replacement therapy have been established by the National Kidney Foundation in their KDOQI Guidelines and are based on recent evidence demonstrating that delaying initiation of renal replacement therapy until patients are malnourished or have severe uremic complications leads to a worse prognosis on dialysis or with transplantation. Because of the interindividual variability in the severity of uremic symptoms and renal function, it is ill-advised to assign an arbitrary urea nitrogen or creatinine level to the need to start dialysis. Moreover, patients may become accustomed to chronic uremia and deny symptoms, only to find that they feel better with dialysis and realize in retrospect how poorly they were feeling before its initiation.

Previous studies suggested that starting dialysis before the onset of severe symptoms and signs of uremia were associated with prolongation of survival. This led to the concept of "healthy" start and is congruent with the philosophy that it is better to keep patients feeling well all along rather than allowing them to become ill with uremia before trying to return them to better health with dialysis or transplantation. Although recent studies have not confirmed a clear association of early-start dialysis with improved patient survival, there is still merit in this approach. On a practical level, advanced preparation may help to avoid problems with the dialysis process itself (e.g., a poorly functioning fistula for hemodialysis or malfunctioning peritoneal dialysis catheter) and thus preempt the morbidity associated with resorting to the insertion of temporary hemodialysis access with its attendant risks of sepsis, bleeding, and thrombosis.

Transplantation of the human kidney is the treatment of choice for advanced chronic renal failure. Worldwide, tens of thousands of these procedures have been performed. When azathioprine and prednisone initially were used as immunosuppressive drugs in the 1960s, the results with properly matched familial donors were superior to those with organs from deceased donors: 75-90% compared with 50-60% graft survival rates at 1 year. During the 1970s and 1980s, the success rate at the 1-year mark for deceased-donor transplants rose progressively. Currently, deceased-donor grafts have an 89% 1-year survival and living-donor grafts have a 95% 1-year survival. Although there has been improvement in long-term survival, it has not been as impressive as the short-term survival, and currently the "average" (t1/2) life expectancy of a living-donor graft is around 20 years and that of a deceased-donor graft is close to 14 years.

Mortality rates after transplantation are highest in the first year and are age-related: 2% for ages 18-34 years, 3% for ages 35-49 years, and 6.8% for ages ≥50-60 years. These rates compare favorably with those in the chronic dialysis population even after risk adjustments for age, diabetes, and cardiovascular status. Occasionally, acute irreversible rejection may occur after many months of good function, especially if the patient neglects to take the prescribed immunosuppressive drugs. Most grafts, however, succumb at varying rates to a chronic process consisting of interstitial fibrosis, tubular atrophy, vasculopathy, and glomerulopathy, the pathogenesis of which is incompletely understood. Overall, transplantation returns most patients to an improved lifestyle and an improved life expectancy compared with patients on dialysis. There are at least 100,000 patients with functioning kidney transplants in the United States.

Both chronic dialysis and renal transplant patients have a higher incidence of death from myocardial infarction and stroke than does the population at large, and this is particularly true in diabetic patients. Contributing factors are the use of glucocorticoids and sirolimus, as well as hypertension. Recipients of renal transplants have a high prevalence of coronary artery and peripheral vascular diseases. The percentage of deaths from these causes has been slowly rising as the numbers of transplanted diabetic patients and the average age of all recipients increase. More than 50% of renal recipient mortality is attributable to cardiovascular disease. In addition to strict control of blood pressure and blood lipid levels, close monitoring of patients for indications of further medical or surgical intervention is an important part of management.

### 3.- Cardiovascular disease

Cardiovascular disease (CVD) is a term for heart and blood vessel diseases, including - among others - ischemic heart disease (being the most common type of CVD in the industrialized countries; this disorder refers to problems with the circulation of the blood to the heart muscle), cerebrovascular disease (refers to a problem with the circulation of the blood in the blood vessels of the brain), and peripheral vascular disease (affecting the circulation primarily in the legs). Subjects with CVD may develop a number of complications (hereinafter referred to as CVD complications) including, but not limited to, myocardial infarction, stroke, angina pectoris, transient ischemic attacks, congestive heart failure, aortic aneurysm and death.

The *Framingham Heart Study* (Kamel WB et al. Am J Cardiol 1988;62:1109-1112., Wilson PWF et al., Circulation 1988;97:1837-1847, Grundy S.M. et al. Circulation 1988;97:1876-1887) was a pioneering study in the development of the concept of risk factors, which is widely used and accepted today.Several independent risk factors have been recognized to be the direct cause of coronary disease and are frequent factors in the population, and their modification reduces the risk of coronary (i.e. cardiovascular) events (Grundy S.C. et al., Circulation 2000;101:e3-e11).The main modifiable risk factors are smoking, high blood pressure, hypercholesterolemia (in particular high LDL-cholesterol) and diabetes mellitus (Circulation 2000;101:e3-e11). The adaptation of all actions according to the absolute risk (the probability that a person develops a coronary disease in a certain period of time) is important, because it allows reach a suitable balance between efficacy, safety and therapy costs. The estimation of the absolute risk requires adding up the contribution of each risk factor and the result is "the determination of the global risk". The *Framingham Heart Study,* as mentioned above, performed a quantitative estimation of the global risk based on the contribution of each risk.

Although traditional risk factors are important predictors of cardiac events in individuals with CKD, the Framingham equations do not accurately weight these risk factors in predicting coronary heart disease events in CKD (J Am Coll Dardiol 2007;50:217-224). Much of this failure is driven by competing events with death as well as the significantly higher cardiac event rate in CKD patients. While it is possible to somewhat improve coronary heart disease prediction in individuals with CKD, particularly in women, both by recalibrating the equations and by assigning different importance to traditional Framingham risk factors, it is likely that future predictive equations with adequate sample size for development and validation of a predictive model in CKD will need to examine both cardiac events and mortality in this high risk population with the goal of identifying shared modifiable risk factors for adverse outcomes.

EP 2 554 679 A1 and EP 2 264 183 A1 concern different patient groups, which also is true for Patrick Linsel-Bitschke et al: "Genetic variations in the arachidonate 5-lipoxygenase-activating protein (ALOX5AP is associated with myocardial infarction in the German population", CLINICAL SCIENCE, vol. 115, no. 10, 1 Noember 2008 (2008-11-01), page 309, WO2012/001613 A1 (FUNDACIO INST DE RECERCA HOSPITAL UNI VALL D HEBRON [ES]; MONTANER VIL) 5 January 2012 (2012-01-05), ANNABEL Z. WANG ET AL: "Association of SNP rs 17465637 on Chromosome 1q41 and rs599839 on 1p13.3 with Myocardial Infarction in an American Caucasian Population", ANNALS OF HUMAN GENETICS, vol. 75, no. 4, 4 April 2011 (2011-04-04), pages 475-482), YAN YU ET AL: "Evaluation of pupulation impact of candidate polymorphisms for coronary heart disease in the Framingham Heart Study Offspring Cohort", BMC PROCEEDINGS, BIOMED CENTRAL LTD, LONDON UK, vol. 3, no Suppl 7,15 December 2009 (2009-12-15), page S118, YUMIKO HIURA ET AL: "Validation of the Association of Genetic Variants on Chromosome 9p21 and 1p41 With Myocardial Infarction in a Japanese Population", CIRCULATION JOURNAL, vol. 72, no. 8, 1 August 2008 (2008-08-01), pages 1213-1217 and EAGLE K A ET AL: "Summaries of Key Journal Articles" JOURNAL OF THE AMERICAN COLLEGE OF CARDIOLOGY, ELSEVIER, NEW YORK, NY, US, vol. 55, no. 9, 2 March 2010 (2010-03-02), pages 948-955.

M RAO ET AL: "Cytokine gene polymorphisms and progression of renal and cardiovascular diseases", KIDNEY INTERNATIONAL, vol. 72, no. 5, 20 June 2007 (2007-06-20), pages 549-556 only comprises very general data.

ARKING D ET AL: "Understanding cardiovascular disease through the lense of genome-wide association studies,", TRENDS IN GENETICS; ELSEVIER SCIENCE PUBLISHERS B.V. AMSTERDAM, NL, vol. 25, no. 9, 1 September 2009 (2009-09-01), pages 387-394, ROBERT CLARKE ET AL: "Genetic Variants Associated with Lp(a) Lipoprotein Level and Coronary Disease", NEW ENGLAND JOURNAL OF MEDICINE, vol. 361, no. 26, 24 December 2009 (2009-12-24), pages 2518-2525, and WO 2008/060618 A2 are again concerned with different patient groups.

Accordingly, there is a need for novel markers, including new genetic markers and combinations thereof that could successfully and advantageously define the cardiovascular risk of a subject suffering from chronic kidney disease and predict who is at high risk of developing cardiovascular disease and/or cardiovascular disease complications such as -but not limited to- fatal or non-fatal myocardial infarction or angina pectoris or stroke or transient ischemic attack or peripheral arteriopathy, in a way that preventive and/or therapeutic measures could be implemented to keep that risk at the lowest possible level.

## SUMMARY OF THE INVENTION

In a first aspect, the invention provides a method which is suitable to solve the limitations of the scales/methods nowadays in use to calculate the cardiovascular risk in patients suffering from chronic kidney disease.

The method provided according to the present invention solves the above mentioned limitations by determining the cardiovascular risk of a subject suffering from chronic kidney disease and predicts his/her risk of developing cardiovascular disease and/or cardiovascular disease complications comprising the steps of determining in a sample isolated from said subject suffering from chronic kidney disease the presence of polymorphisms, wherein said polymorphisms are at positions 27 within the nucleic acid sequences of SEQ ID NO:1 to 8, wherein the presence at position 27 of a C in SEQ ID NO:1, C in SEQ ID NO:2, T in SEQ ID NO:3, C in SEQ ID NO:4, C in SEQ ID NO:5, A in SEQ ID NO:6, T in SEQ ID NO:7, and G in SEQ ID NO:8, which correspond to db SNP accession number rs17465637, rs6725887, rs9818870, rs12526453, rs1333049, rs501120, rs9982601, rs10455872, respectively, as futher defined in the claims.

These sequences may thus also be used for the purposes of the present invention, as the rs that is in strong linkage disequilibrium with any of those rs mentioned, as described herein.

In any of said rs sequences that are in strong linkage disequilibrium with an rs sequence mentioned herein, the polymorphism may, for example, be at position 27. The skilled person may access rs sequences on the NCBI SNP database ("dbSNP", http://www.ncbi.nlm.nih.gov/snp).

In the invention the combination of SEQ ID NO: 1-11, is used for the above method.

In another aspect, the invention relates to a method of determining the cardiovascular risk of a subject suffering from chronic kidney disease and who has suffered one or more cardiovascular event to predict the risk of developing cardiovascular disease and/or cardiovascular disease complications comprising the steps of determining in a sample isolated from said subject the presence of a polymorphisms wherein said polymorphism is at positions 27 in the nucleotide sequences of SEQ ID NO:1 to 11, or said polymorphism is in a sequence that is in strong linkage disequilibrium with the sequence of any of SEQ ID NO:1 to 11, wherein said polymorphism at said position 27 of a C in SEQ ID NO:1, C in SEQ ID NO:2, T in SEQ ID NO:3, C in SEQ ID NO:4, C in SEQ ID NO:5, A in SEQ ID NO:6, T in SEQ ID NO:7, G in SEQ ID NO:8, A in SEQ ID NO:9, A in SEQ ID NO:10 and/or G in SEQ ID NO:11, which correspond to db SNP accession number rs17465637, rs6725887, rs9818870, rs12526453, rs1333049, rs501120, rs9982601, rs10455872, rs10507391, rs9315051, and/or rs17222842, respectively, or the presence of a polymorphism within the sequence of any other rs that is in strong linkage disequilibrium with any of those rs mentioned is indicative of a risk of having an adverse cardiovascular disease or disorder, as further defined in claim 1.

The disclosure further provides a method for identifying a subject suffering from chronic kidney disease in need of early and/or a more aggressive cardiovascular therapy or in need of prophylactic cardiovascular therapy comprising the steps of determining in a sample isolated from said subject the presence in at least one allele of a polymorphism, wherein said polymorphism is at positions 27 within the nucleic acid sequenceof any of SEQ ID NO:1 to 11,, or said polymorphism is in a sequence that is in strong linkage disequilibrium with any of SEQ ID NO:1 to 11, wherein the presence at position 27 of a C in SEQ ID NO:1, C in SEQ ID NO:2, T in SEQ ID NO:3, C in SEQ ID NO:4, C in SEQ ID NO:5, A in SEQ ID NO:6, T in SEQ ID NO:7, G in SEQ ID NO:8, A in SEQ ID NO:9, A in SEQ ID NO:10 and/or G in SEQ ID NO:11, which correspond to db SNP accession number rs17465637, rs6725887, rs9818870, rs12526453, rs1333049, rs501120, rs9982601, rs10455872, rs10507391, rs9315051, and/or rs17222842, respectively, or the presence of a polymorphism within the sequence of any other rs that is in strong linkage disequilibrium with any of those rs mentioned is indicative of being in need of early and/or a more aggressive cardiovascular therapy or in need of prophylactic cardiovascular treatment.

In an advantageous embodiment, it is possible to use the combination of SEQ ID NO: 1 - 7, or 1 - 8, or 1 - 11, for the above method.

A "prophylactic" therapy means a therapy which is started before symptomatic onset of a disease.

An "early or a more aggressive" therapy means a therapy which is started at an early stage of a disease or with higher amounts or higher frequency than on average due, for instance, to be at a higher risk of developing CHD when using this invention and therefore therapeutic objectives have to be adapted according to guidelines. These new objectives would require a more aggressive therapy.

In another aspect, the disclosure relates to a method of treatment of a patient suffering from chronic kidney disease and suffering from a cardiovascular disease with a cardiovascular therapy wherein the patient is selected for said therapy based on the presence in a sample isolated from said subject of a polymorphism, wherein said polymorphism is at position 27 in the nucleotide sequences of SEQ ID NO:1 to 11, or said polymorphism is in a sequence that is in strong linkage disequilibrium with the sequence of any of SEQ ID NO:1 to 11, wherein the presence at said position 27 of a C in SEQ ID NO:1, C in SEQ ID NO:2, T in SEQ ID NO:3, C in SEQ ID NO:4, C in SEQ ID NO:5, A in SEQ ID NO:6, T in SEQ ID NO:7, G in SEQ ID NO:8, A in SEQ ID NO:9, A in SEQ ID NO:10 and/or G in SEQ ID NO:11, which correspond to db SNP accession number rs17465637, rs6725887, rs9818870, rs12526453, rs1333049, rs501120, rs9982601, rs10455872, rs10507391, rs9315051, and/or rs17222842, respectively, or the presence of a polymorphism within the sequence of any other rs that is in strong linkage disequilibrium with any of those rs mentioned indicates that the patient is selected for said therapy.

In an advantageous embodiment, it is possible to use the combination of SEQ ID NO: 1 - 7, or 1- 8, or 1 -11, for the above method.

In another aspect, the diclosure relates to a method of determining the cardiovascular risk of a subject suffering from chronic kidney disease at any of the stages 1 to 5 of chronic kidney disease and/or under replacement therapy with dialysis (hemodialysis or peritoneal dialysis) and/or with a kidney transplant to predict who is at high risk of developing cardiovascular disease and/or cardiovascular disease complications and/or of determining the response to a cardiovascular therapy comprising the steps of determining in a sample isolated from said subject the presence of a polymorphism, wherein said polymorphism is at positions 27 within the nucleic acid sequences of SEQ ID NO:1 to 11, or said polymorphism is in a sequence that is in strong linkage disequilibrium with the sequence of any of SEQ ID NO:1 to 11, wherein the presence at position 27 of a C in SEQ ID NO:1, C in SEQ ID NO:2, T in SEQ ID NO:3, C in SEQ ID NO:4, C in SEQ ID NO:5, A in SEQ ID NO:6, T in SEQ ID NO:7, G in SEQ ID NO:8, A in SEQ ID NO:9, A in SEQ ID NO:10 and/or G in SEQ ID NO:11 which correspond to db SNP accession number rs17465637, rs6725887, rs9818870, rs12526453, rs1333049, rs501120, rs9982601, rs10455872, rs10507391, rs9315051, and/or rs17222842, respectively, or the presence of a polymorphism within the sequence of any other rs that is in strong linkage disequilibrium with any of those rs mentioned is indicative of an increased risk of having an adverse cardiovascular disease or disorder or of a low response to a cardiovascular therapy.

In an advantageous embodiment, it is possible to use the combination of SEQ ID NO: 1 - 7, or 1 - 8, or 1 - 11, for the above method.

In further aspects, the invention relates to methods for the determination of the probability of an individual suffering from CKD of presenting a fatal or non-fatal myocardial infarction or angina in a 10 year period based on the presence of one or more of the polymorphisms mentioned above in combination with one or more clinical/biochemical risk factors, wherein the relative contribution of each polymorphism or risk factor to the probability is corrected using the hazard ratios The hazard ratio is a measure of how often a coronary event happens in the group with each of the polymorphisms or risk factors compared to how often it happens in the group withouth each of the polymorphisms or risk factors, over time).

In an advantageous embodiment, it is possible to use the combination of SEQ ID NO: 1 - 11, for the above method.

"Improved cardiovascular risk assessment" in the context of this application should be understood as a prediction of the probability to develop a cardiovascular event that fits better than the risk assessment done by scales/methods nowadays in use, such as but not limited to Framingham risk score, adapted Framingham risk score (such but not limited to Regicor), Score, HeartScore, Procam, Reynolds, QRisk, with the number of events that a particular patient has suffered (within the context of a retrospective study) or will suffer, and the MACE and all-cause mortality calculator construction using the ALERT extension trial data that includes as variable of interest age, previous coronary heart disease, smoking, serum creatinine, diabetes mellitus, LDL-cholesterol (for MACE only), total time on renal replacement therapy (for MACE only), and number of transplants (for mortality only), calculator that can be accessed at http://www.anst.uu.se/insov254/calculator/ (Transplantation 2013;95:142-147). The improvement can be measured as an increase in the area under the ROC curve, measures as a higher c statistic value calculated according to Acad Radiol 1997;4:49-58.,, the net reclassification improvement and/or integrated discrimination improvement calculated according to Statist Med 2008;27:157-172.

The validation of the markers included in the present invention have been done following the recommendations of the AHA (Circulation 2009;119:2408-2416).

**Table 1. Recommendations for Reporting of Novel Risk Markers**

| | | |
|---|---|---|
| 1. | Report the basic study design and outcomes in accord with accepted standards for observational studies[4] | |
| 2. | Report levels of standard risK factors and the results of risk model using these established factors | |
| 3. | Evaluate the novel marker risk the population and report : | |
| | a. | Relate risK odds ratio, or Hazard ratio conveyed by the novel marker alone, with the associated confidence limits and *P* value |
| | b. | Relative risk, odds ratio, or hazard ratio for novel marker after statistical adjustment tor established risk factors, with the associated confidence limits and *P* value |
| | c. | *P* value for addition of the novel marker to a model that contains the standard risk markers |
| 4. | Report the discrimination of the new marker: | |
| | a. | C-index and its confidence limits for model with established risk markers |
| | b. | C-index and its confidence limits for model including novel marker and established risk markers |
| | c. | integrated discrimination index, discrimination slope, or binary $R^2$ for the model with and without the novel risk marker |
| | d. | Graphic or tabular display of predicted risk in cases and noncases separately, before and after inclusion of the new marker |
| 5. | Report the accuracy of the new marker: | |
| | a. | Display observed vs expected event rates across the range of predicted risk for models without and with the novel risk marker |
| | b. | Using generally recognized risk thresholds, report the number of subjects reclassified and the event rates in the reclassified groups |

"Improved cardiovascular risk assessment" in the context of this application is used interchangeably with "refined cardiovascular risk assessment".

The present methods, as described throughout this application, are in a preferred embodiment all carried out ex *vivo.*

In a preferred embodiment the presence of the following alleles of polymorphisms is determined: polymorphisms at positions 27 within specific nucleic acid sequences in particular the presence at position 27 of a C in SEQ ID NO:1, C in SEQ ID NO:2, T in SEQ ID NO:3, C in SEQ ID NO:4, C in SEQ ID NO:5, A in SEQ ID NO:6, T in SEQ ID NO:7, G in SEQ ID NO:8, A in SEQ ID NO:9, A in SEQ ID NO:10 and G in SEQ ID NO:11 with db SNP accession number rs17465637, rs6725887, rs9818870, rs12526453, rs1333049, rs501120, rs9982601, rs 10455872, rs10507391, rs9315051, and/ or rs17222842, respectively. The presence of a polymorphism, or an allele of a polymorphism, may also be determined in any other rs that is in strong linkage disequilibrium with any of those rs or SEQ ID NOs mentioned as far as encompassed by the claims. For example, presence of a polymorphism, or an allele of a polymorphism, may be determined at position 27 of any such other rs that is in strong linkage disequilibrium with any rs or SEQ ID NOs mentioned herein.

In a preferred embodiment the presence of the following alleles of polymorphisms is determined: polymorphisms at positions 27 within specific nucleic acid sequences in particular the presence at position 27 of a C in SEQ ID NO:1, C in SEQ ID NO:2, T in SEQ ID NO:3, C in SEQ ID NO:4, C in SEQ ID NO:5, A in SEQ ID NO:6, T in SEQ ID NO:7, G in SEQ ID NO:8, A in SEQ ID NO:9, A in SEQ ID NO:10 and G in SEQ ID NO:11 with db SNP accession number rs17465637, rs6725887, rs9818870, rs12526453, rs1333049, rs501120, rs9982601, rs10455872, rs10507391, rs9315051, and/or rs17222842, respectively. The presence of a polymorphism, or an allele of a polymorphism, may also be determined , (e.g. at position 27) in any other rs in strong linkage disequilibrium with any of those rs or SEQ ID NOs mentioned, as far as encopassed by the claims. The A in SEQ ID NO:9, A in SEQ ID NO:10 and G in SEQ ID NO:11 with db SNP accession number rs10507391, rs9315051, and rs17222842, respectively (or the polymorphism in any other rs in strong linkage disequilibrium with any of those rs mentioned), are together forming the haplotype B ALOX5AP and being considered as one risk genetic component in addition to the other 8 sequences.

These embodiments, i.e. specific combinations of SNPs, in particular the combination of SEQ ID NO 1 - 11, are preferred embodiments of all aspects of this invention described below.

In another aspect, the invention relates to methods for the estimation of the probability (known as cardiovascular risk) of an individual suffering from chronic kidney disease of presenting a fatal or non-fatal myocardial infarction, or angina, or stroke, or transient ischemic attack or peripheral arteriopathy in a ten year period and/or long-life period based on the presence of the polymorphisms mentioned above in combination with one or more clinical and/or biochemical risk factors, wherein the relative contribution of the polymorphisms is given as a genetic score risk.

In another aspect, the invention relates to methods for the estimation of the probability (known as cardiovascular risk) of an individual suffering from chronic kidney disease of presenting a fatal or non-fatal myocardial infarction, or angina, or stroke, or transient ischemic attack or peripheral arteriopathy in a ten year period and/or long-life period based on the presence of the polymorphisms mentioned above, preferably in combination with one or more clinical and/or biochemical risk factors, wherein the relative contribution of the polymorphisms is given as a genetic score risk. The use of the present invention may imply a reclassification of the cardiovascular risk in comparison to the classical way of estimating the risk in patients suffering from chronic kidney disease (e.g. Framingham, Regicor, Score, MACE or total mortality risk calculators among others) where the risk is estimated without the consideration of the genetic and/or clinic/biochemical data considered in the present invention. Reclassification means that by considering the present invention, the new risk can be lower or higher than that calculated by classic means.

"Cardiovascular event" in the context of this application is used interchangeably with "cardiovascular complication".

"AHA" in the context of this application should be understood as American Heart Association.

"GWAS" in the context of this application should be understood as genome-wide association studies.

The term "disease" and "disorder" shall be interpreted in the context of this application interchangeably.

The term "chronic kidney disease" is meant to define a Kidney damage for ≥3 months, as defined by structural or functional abnormalities of the kidney, with or without decreased Glomerular Filtration Rate (GFR), that can lead to decreased GFR, manifest by either pathological abnormalities or markers of kidney damage, including abnormalities in imaging test or a GFR <60 mL/min/11.73 $m^2$ for ≥ 3 months, with or without kidney damage (Am J Kidney Dis 39:S1-S266, 2002 suppl 1).

In another aspect, the invention relates to methods for the reclassification of the probability (known as cardiovascular risk) of an individual suffering from chronic kidney disease classified as having a moderate risk to suffer a cardiovascular event (fatal or non-fatal myocardial infarction, or angina, or stroke, or transient ischemic attack or peripheral arteriopathy) in a ten year period and/or long-life period according to the methods nowadays in use based on the presence of one or more of the polymorphisms mentioned above in combination with one or more conventional risk factors, wherein the relative contribution of the polymorphisms is given as a genetic score risk.

In further aspects, the invention relates to methods for the determination of the probability of an individual suffering from chronic kidney disease of presenting a fatal or non-fatal myocardial infarction or angina pectoris or stroke or transient ischemic attack or peripheral arteriopathy in a ten year period or in a long-life period based on the presence of one or more of the polymorphisms mentioned above in combination with one or more conventional risk factors, wherein the relative contribution of the polymorphisms is given as a genetic score risk,

In further aspects, the invention relates to methods for the determination of the probability of an individual suffering from chronic kidney disease, and having suffered from one or more non-fatal myocardial infarction or angina pectoris or stroke or transient ischemic attack or peripheral arteriopathy of presenting a fatal or non-fatal myocardial infarction or angina pectoris or stroke or transient ischemic attack or peripheral arteriopathy in a ten year period or in a long-life period based on the presence of the polymorphisms mentioned above, preferably in combination with one or more conventional risk factors, wherein the relative contribution of the polymorphisms is given as a genetic score risk.

In a further aspect, the specification relates to a computer program or a computer-readable media containing means for carrying out any of the methods of the invention.

In yet a further aspect, the invention relates to a kit comprising reagents for detecting the identity of the nucleotide at position 27 within the nucleic acid sequences of SEQ ID NO:1 to 11, as further defined in the claims. In yet a further aspect, the invention relates to a kit comprising reagents for detecting the identity of the nucleotide at position 27 within a nucleic acid sequence selected from the group of SEQ ID NO:1 to 8, and the following SEQ ID NO: 9, 10, and 11 being considered as haplotype B ALOX5AP and the alleles AAG in those SEQ being considered as a single risk allele.

In a further aspect, the invention relates to a computer program or a computer-readable media containing means for carrying out any of the methods of the invention.

The invention is also further defined by the figures, wherein Figure 1 shows an overview of preferred genes and SNPs and Figure 2 shows how CKD population had a higher cardiovascular and coronary risk than a non-CKD general Spanish population (Rev Esp Cardiol 2003;56(3):253).

**DETAILED DESCRIPTION OF THE INVENTION**

The authors of the present invention have identified a series of single nucleotide polymorphism (SNP) markers which, when used in combination, are associated with a risk of CVD and/or with a risk of CVD complications including, but not limited to, myocardial infarction, stroke, angina pectoris, and peripheral arteriopathy in patients suffering from chronic kidney disease. These polymorphic markers are superior to the polymorphisms or combination of polymorphisms currently in use.

The authors of the present invention have solved the problem identified above in the scaies/methods/equations in use nowadays for the calculation of the risk of a subject suffering from chronic kidney disease to develop cardiovascular disease, cardiovascular events and/or cardiovascular complications including, but not limited to, fatal- and non-fatal myocardial infarction, stroke, angina pectoris, transient ischemic attacks, and peripheral arteriopathy.

The present application thus also pertains to a method to overcome the limitations of the existent scales/methods/equations, which do not accurately predict a cardiovascular disease in patients suffering from chronic kidney disease.

The present application thus also pertains to a method to overcome the limitations of the existent scales/methods/equations, which still allow a significant number of cardiovascular events to occur in subjects suffering from chronic kidney disease with only a calculated intermediate risk, using the tools nowadays in use for cardiovascular risk.

The present application thus also pertains to a method to overcome the limitations of the scales/methods/equations, which do not accurately predict a cardiovascular disease in patients suffering from chronic kidney disease in particular those who already have suffered one or more cardiovascular diseases.

The present application overcomes the above-described limitations of the scales/methods/equations used nowadays to calculate the cardiovascular risk by providing a method to accurately calculate the cardiovascular risk in patients suffering from chronic kidney disease. A particular combination (as described above) of genetic markers is used. Even more preferred and advantageous is the combination as listed in table 1 (see Figure 1), selected and evaluated by the inventors after a complex and genuine analysis of thousands of possible markers. Of the different possibilities to construct a genetic risk score (GRS), the inventors have been successful to identify a particular one, whereby this combination provided the best possible results. To calculate the genetic risk punctuation, the accumulated number of risk alleles from those SNPs listed in table 1 that are present in each individual is considered. For each of the variants studied, every individual can have 0, 1 or 2 alleles of risk. On having calculated the summatory of risk alleles accumulated in the different sets of the selected variants (n=9, or 8), for each individual, a score that could go from 0 to 18, or 16, respectively, was given. The inventors have generated new algorithms for cardiovascular risk estimation. This innovative strategy allows accurate cardiovascular risk estimation and further allows reclassification of patients with excellent net reclassification improvement values.

Preferred aspects are set out as follows:

1. A method for a cardiovascular risk assessment in a subject comprising the steps of determining in a sample isolated from said subject suffering from chronic kidney disease the presence of a polymorphism, wherein said polymorphism is at positions 27 within the nucleic acid sequences of SEQ ID NO:1 to 8, or said polymorphism is in a sequence that is in strong linkage disequilibrium with the sequence of any of SEQ ID NO:1 to 8, wherein the presence at position 27 of a C in SEQ ID NO:1, C in SEQ ID NO:2, T in SEQ ID NO:3, C in SEQ ID NO:4, C in SEQ ID NO:5, A in SEQ ID NO:6, T in SEQ ID NO:7, and/or G in SEQ ID NO:8, which correspond to db SNP accession number rs17465637, rs6725887, rs9818870, rs12526453, rs1333049, rs501120, rs9982601, rs10455872, respectively, or the presence of a polymorphism within the sequence of any other rs that is in strong linkage disequilibrium with any of those rs mentioned, is indicative of a risk of having a cardiovascular event.
2. The method of item 1, wherein in addition the presence at position 27 of an A in SEQ ID NO: 9, A in SEQ ID NO:10, and/or G in SEQ ID NO:11 with db SNP accession no. rs10507391, rs9315051, and/or rs17222842, respectively, or any other rs in strong linkage disequilibrium with any of those rs mentioned is determined.
3. The method of item 1 and/or 2 above, for a reclassification of a subject suffering from chronic kidney disease to an improved risk assessment compared to that obtained using the scales/methods for such risk estimation such as, but not limited to Framingham, Regicor, Score, Procam. Qrisk, MACE, total mortality risk calculator.
4. The method of any of item 1-3 above for identifying a subject suffering from chronic kidney disease in need of cardiovascular therapy or in need of preventive cardiovascular therapy/measurements for a cardiovascular event or in need of specific therapeutic objectives.

5. The method of any one of items 2-4 above, wherein the latter three SEQ (SEQ ID NO 9 -11) and rs are forming the haplotype B ALOX5AP and are considered as one risk genetic component in addition to the other 8 sequences.

6. The methods of any one of the items 1-5 above for the determination of the probability of an individual suffering from chronic kidney disease and having suffered from one or more non-fatal myocardial infarction or angina pectoris or stroke or transient ischemic attack or peripheral arteriopaty of presenting a fatal or non-fatal myocardial infarction or angina pectoris or stroke or transient ischemic attack or peripheral arteriopathy.

7. The method of any one of items 1-6 above to establish the therapeutical objectives of preventive and/or therapeutical treatments for a patient suffering from chronic kidney disease and having a cardiovascular event or suspected of having a predisposition for a cardiovascular event wherein the patient and/or the therapeutical objectives are selected for said therapy based on the presence in a sample isolated from said subject of the polymorphism defined above.

8. The method of any one of items 1-7 above, for determining the probability of an individual suffering from chronic kidney disease of presenting a fatal or non-fatal myocardial infarction or angina in a 10 year period based on the presence of 1 to P clinical and/or biochemical risk factors and 1 to J polymorphism at positions 27 in the above nucleotides sequences of SEQ ID NO:1 to 11, using the formula 1a:

$$prob(event_i \mid CRF_{p,i}, SNP_{j,i}) = 1 - \hat{S}^{\exp\left[\sum_{p=1}^{P}\beta_{CRF_p}*CRF_{p,i} + \sum_{j=1}^{J}\beta_{SNP_j}*SNP_{j,i} - \sum_{p=1}^{P}\beta_{CRF_p}*\overline{CRF_p} - \sum_{j=1}^{J}\beta_{SNP_j}*\overline{SNP_j}\right]}$$

wherein,

$prob(event_i|CRF_{p,i}, SNP_{j,i})$ is the probability of presenting a coronary event given a combination of coronary risk factors (CRF) and genetic characteristics ($SNP_{j,i}$),

- $event_i$: coronary event (fatal and nor-fatal myocardial infarction or angina) in a 10-year period for an individual "i",
- $CRF_{p,i}$: value of each coronary risk factor "p" included in the equation for an individual "i". The list of the coronary risk factors included in the model is shown in table A,
- $SNP_{j,i}$: number of risk alleles (0,1,2) for a specific genetic variant "j" included in the equation for an individual "i". The variants currently included in the model are shown in table B,

$\hat{S}$ is the mean survival free of coronary events at the population. This survival will be adapted to the regional or national rates,

exp; natural exponentiation $\sum_{p=1}^{p}$ is the summatory function along the P classical risk factors,

$\beta_{CRF_p}$ is the logarithm of hazard ratio corresponding to the coronary risk factor "p" as shown in table A,

$CRF_{p.i}$ is the value of each coronary risk factor "p" included in the equation for an individual "i", $\sum_{J=1}^{J}$ is the summatory function along the J genetic variants,

$\beta_{SNP_j}$ is the logarithm of hazard ratio corresponding to the genetic variant "j" as shown in table B,

$SNP_{j,i}$ is the number of risk alleles (0, 1, 2) for a specific genetic variant "j" included in the equation for an individual "i",

$\overline{CRF_p}$ is the average value for the clinical/biochemical risk factor "p" in the population,

$\overline{SNP_j}$ is the average risk allele number of copies for genetic variant "j" in the population.

9. The method of any one of items 1-7 above for determining the probability of an individual suffering from chronic kidney disease of presenting a fatal or non-fatal myocardial infarction or angina in a 10 year period based on the presence of 1 to P different clinical/biochemical risk factors and 1 to J different genetic variants wherein said genetic variant is the above polymorphism at positions 27 in the nucleotide sequences of SEQ ID NO:1 to 11, using the formula 1b:

$$prob(event_i \mid CRFp,i, GRS_i =$$

$$1 - \hat{S}^{\exp\left[\sum_{p=1}^{p}\beta_{CRF_p} * CRFp,i + \beta_{GRS} * GRS_i - \sum_{p=1}^{p}\beta_{CRF_p} * \overline{CRF_p} - \beta_{GRS} * \overline{GRS}\right]}$$

wherein

$prob(event_i|CRF_{p,i}, GRS_i)$ is the probability of presenting a coronary event given a combination of coronary risk factors ($CRF_{p,i}$) and genetic characteristics ($GRS_i$),

- $event_i$: coronary event (fatal and nor-fatal myocardial infarction or angina) in a 10-year period for an individual "i",
- $CRF_{p,i}$: value of each coronary risk factor "p" included in the equation for an individual "i". The list of the coronary risk factors included in the model is shown in table A,
- $GRS_i$: genetic risk score defined as the weighted number of risk alleles (0,1,2) for the genetic variants included in the equation for an individual "i". The variants included in the genetic risk score are shown in table A. The weights are proportional to the betas of each SNP included in the score (Shown in table B), and the range of the GRS goes from 0 to twice the number of SNPs included in the score,

$\hat{S}$ : mean survival free of coronary events in the population,
exp: natural exponentiation,

$$\sum_{p=1}^{p}\beta_{CRF_p} * CRFp,i$$

where $\sum_{p=1}^{p}$ is the summatory function along the "p" clinic/biochemical risk factors,
$\beta_{CRF_p}$ is the logarithm of hazard ratio corresponding to the clinical/biochemical risk factor "p". The values of the $\beta$ for each coronary risk factor "p" are shown in table A,
$CRF_{p,i}$ is the value of each coronary risk factor "p" included in the equation for an individual "i",
$\beta_{GRS}$ is the logarithm of the hazard ratio corresponding to one unit increase in the value of the genetic risk score. The value of this $\beta_{GRS}$ is 0.104 with a range of values going from 0.010 to 0.500,
$GRS_i$ is the value of the genetic risk score for an individual "i",
$\overline{CRF_p}$ is the average value for the clinical/biochemical risk factor "p" in the population. This average value will be adapted to the regional or national prevalence,
$\overline{GRS}$ is the mean value of the genetic risk score in the population;
and other variables are defined as in item 8.

10. A method as defined in any of the items 1 to 9 wherein the cardiovascular event is selected from the group of fatal or non-fatal myocardial infarction, stroke, angina pectoris, transient ischemic attacks, peripheral arterial disease or a combination thereof.
11. A method as defined in any of items 1 to 10 further comprising determining one or more cardiovascular disease or disorder risk factor(s) selected from the group consisting of age, race, sex, body mass index, systolic blood pressure, diastolic blood pressure, smoking status and history, total cholesterol, low density lipoprotein (LDL)- or high density lipoprotein (HDL)-cholesterol level, triglycerides, dyslipemia history, history of heart failure, previous coronary heart disease, previous coronary heart disease, diabetes mellitus, glycemia, glycated hemoglobin, hemoglobin A1c, glomerular filtration, kidney disease status (CKD status 1-4, End Stage Renal Disease (ESRD) in renal replacement therapy or kidney transplantation), hypertension, renal insufficiency and its status (1 to 5), chronic kidney disease and its status (pre-dialysis, dialysis, transplantation, transplantation failure), total time on renal replacement therapy, number of transplants, left ventricular hypertrophy, alcohol consumption, physical activity practice, diet and family history of cardiovascular or coronary disease, creatinine, calcium, phosphorus, parathormone, albumin, and 24 hours proteinuria.

12. The method according to any one of items 1 to 11 wherein the sample is an oral tissue sample, scraping, or wash or a biological fluid sample, preferably saliva, urine or blood.

13. The method according to any one or more of items 1 to 12 wherein the presence or absence of the polynucleotide is identified by amplifying or failing to amplify an amplification product from the sample, wherein the amplification product is preferably digested with a restriction enzyme before analysis and/or wherein the SNP is identified by hybridizing the nucleic acid sample with a primer labelled which is a detectable moiety.

14. A method as defined in items 1 to 13 wherein a plurality of classical risk factors "p" are used, said plurality being selected from the group of:

- Sex, age, Total cholesterol, HDL-cholesterol, blood pressure, diabetes and smoking,
- Age, LDL-cholesterol, HDL-cholesterol, triglycerides, systolic blood pressure, family history of myocardial infarction and diabetes,
- Sex, Log(age/10), total cholesterol/HDL-cholesterol, body mass index, family history of premature CVD, smoking, Townsend score of output area, systolic blood pressure, treatment for hypertension and interaction Systolic Blood Pressue (SBP)*Hypertension (HTN) treatment,
- Kidney disease status (CKD stages 1-4, ESDR in renal replacement therapy or kidney transplantation), age, sex, HDL-cholesterol, diabetic condition, hypertension condition, hemoglobin A1c,
- Age, previous coronary heart disease, smoking, serum creatinine, diabetes mellitus, LDL-cholesterol, total time on renal replacement therapy,
- Age, previous coronary heart disease, smoking, serum creatinine, diabetes mellitus, LDL-cholesterol, total time on renal replacement therapy, number of transplants.

15. A method as defined in items 1 - 14 wherein the probability is determined for the period from 15 to 85 years of age of the subject.

16. A method as defined in items 1 - 14 wherein the probability is determined for the period from 35 to 75 years of age of the subject.

17. A method as defined in items 1 - 14 wherein the probability is determined for the period from the actual age of the subject and until the age of 75 years of age of the subject.

18. A method as defined in items 1 - 17, wherein said other rs in strong linkage disequilibrium with any of those rs mentioned is (i) rs1746048, which is represented by SEQ ID NO:48 and is in strong linkage disequilibrium with rs501120, or is (ii) rs2133189, which is represented by SEQ ID NO:49 and is in strong linkage disequilibrium with rs17465637.

19. A computer program or a computer-readable media containing means for carrying out a method as defined in any of items 1 to 18.

20. A kit comprising reagents for detecting the identity of the nucleotide at position 27 within a nucleic acid sequence selected from the group of SEQ ID NO:1 to 11.

21. A kit comprising reagents for detecting the identity of the nucleotide at position 27 within a nucleic acid sequence selected from the group of SEQ ID NO: 1 to 8.

22. A kit comprising reagents for detecting the identity of the nucleotide at position 27 within a nucleic acid sequence selected from the group of SEQ ID NO: 1 to 7.

23. A kit as defined in item 20 or 22 which comprises one or more primer pairs specific for the amplification of a region comprising at least position 27 within a nucleic acid sequence of SEQ ID NO: 1 to 7, or SEQ ID NO: 1 to 8, or SEQ ID NO: 1 - 11.

24. A kit as defined in item 20 or 23 wherein the selected sequences are SEQ ID NO:1, SEQ ID NO:2, SEQ ID NO: 3, SEQ ID NO:4 , SEQ ID NO:5, SEQ ID NO:6, SEQ ID NO:7, SEQ ID NO:8, SEQ ID NO:9, SEQ ID NO:10, and SEQ ID NO:11, with db SNP accession number rs17465637, rs6725887, rs9818870, rs12526453, rs1333049, rs501120, rs9982601, rs10455872, rs10507391, rs9315051, and rs17222842, respectively, or any other rs in strong linkage disequilibrium with any of those rs mentioned.

25. A kit as defined in item 21 or 23, wherein the selected sequences are SEQ ID NO: 1, SEQ ID NO:2, SEQ ID NO:3, SEQ ID NO:4, SEQ ID NO:5, SEQ ID NO:6, SEQ ID NO:7, and SEQ ID NO:8, with db SNP accession number rs17465637, rs6725887, rs9818870, rs12526453, rs1333049, rs501120, rs9982601, and rs10455872, respectively or any other rs in strong linkage disequilibrium with any of those rs mentioned.

26. A kit as defined in item 22 or 23, wherein the selected sequences are SEQ ID NO: 1, SEQ ID NO:2, SEQ ID NO:3, SEQ ID NO:4, SEQ ID NO:5, SEQ ID NO:6, and SEQ ID NO:7, with db SNP accession number rs17465637, rs6725887, rs9818870, rs12526453, rs1333049, rs501120, and rs9982601, respectively or any other rs in strong linkage disequilibrium with any of those rs mentioned.

27. A kit as defined in items 24 - 26, wherein said other rs in strong linkage disequilibrium with any of those rs mentioned is (i) rs1746048, which is represented by SEQ ID NO:48 and is in strong linkage disequilibrium with rs501120, or is (ii) rs2133189, which is represented by SEQ ID NO:49 and is in strong linkage disequilibrium with rs17465637.

28. A set of probes/primers, selected from the following group:

SEQ ID NO:12 and 13 (rs1333049), and
SEQ ID NO:14 and 15 (rs1333049);
SEQ ID NO:16 and 17 (rs10455872) and
SEQ ID NO:18 and 19 (rs10455872);
SEQ ID NO:20 and 21 (rs6725887) and
SEQ ID NO:22 and 23 (rs6725887);
SEQ ID NO:24 and 25 (rs9818870) and
SEQ ID NO:26 and 27 (rs9818870);
SEQ ID NO:28 and 29 (rs10507391) and
SEQ ID NO:30 and 31 (rs10507391);
SEQ ID NO:32 and 33 (rs9982601) and
SEQ ID NO:34 and 35 (rs9982601);
SEQ ID NO:36 and 37 (rs9315051) and
SEQ ID NO:38 and 39 (rs9315051);
SEQ ID NO:40 and 41 (rs12526453) and
SEQ ID NO:42 and 43 (rs12526453);
SEQ ID NO:44 and 45 (rs17222842) and
SEQ ID NO:46 and 47 (rs17222842);
SEQ ID NO: 50 and 51 (rs17465637) and
SEQ ID NO: 52 and 53 (rs17465637);
SEQ ID NO: 54 and 55 (rs501120) and
SEQ ID NO: 56 and 57 (rs501120);
SEQ ID NO: 58 and 59 (rs2133189) and
SEQ ID NO: 60 and 61 (rs2133189);
SEQ ID NO: 62 and 63 (rs1746048) and
SEQ ID NO: 64 and 65 (rs1746048);
SEQ ID NO: 66 and 67 (rs2133189) and
SEQ ID NO: 68 and 69 (rs2133189).

All values as obtained in the function(s) will be adapted to the regional or national prevalence, if necessary. The inventive combination of SNPs for all methods and kits as described herein are thus the combination of the SNPs of SEQ ID NO: 1-11.

The terms "polymorphism" and "single nucleotide polymorphism" (i.e. SNP) are used herein interchangeably and relate to a nucleotide sequence variation occurring when a single nucleotide in the genome or another shared sequence differs between members of species or between paired chromosomes in an individual. A SNP can also be designated as a mutation with low allele frequency greater than about 1% in a defined population. Single nucleotide polymorphisms according to the present application may fall within coding sequences of genes, noncoding regions of genes or the intronic regions between genes.

The list of polymorphisms which are used in this method of the present invention is given in Table 1, included herewith as Fig. 1, and are the SNPs of SEQ ID Nos 1-11.

Herein, a strong linkage disequilibrium may be defined by the $r^2$ value. Linkage disequilibrium is a characterization of the haplotype distribution at a pair of loci. It describes an association between a pair of chromosomal loci in a population. The $r^2$ value is considered particularly suitable to describe linkage disequilibrium.

The $r^2$ measure of linkage disequilibrium is defined as

$$r^2(p_a, p_b, p_{ab}) = \frac{(p_{ab} - p_a p_b)^2}{p_a(1-p_a)p_b(1-p_b)}, \quad (1)$$

where $p_{ab}$ is the frequency of haplotypes having allele *a* at locus 1 and allele *b* at locus 2 (Hill & Robertson. 1968). As the square of a correlation coefficient. $r^2$ ($p_a$, $p_b$, $p_{ab}$) can range from 0 to 1 as $p_a$, $p_b$ and $p_{ab}$ vary.

"Hill & Robertson, 1968" is Theor Appl Genetics 1968;38:226-231.

A strong linkage disequilibrium is one with an r2 value of more than 0.7, preferably more than 0.8, more preferred more than 0.9., including e.g. $r^2$ values of 1.

The term "cardiovascular disease or disorder", as used herein, includes diseases affecting the heart or blood vessels or both, or diseases which are associated with the cardiopulmonary and circulatory systems including - but not limited to - ischemia, angina pectoris, edematous conditions, artherosclerosis, Coronary Heart Disease, LDL oxidation, adhesion of monocytes to endothelial cells, foam-cell formation, fatty-streak development, platelet adherence, and aggregation, smooth muscle cell proliferation, reperfusion injury, high blood pressure, thrombotic disease, arrhythmia (atrial or ventricular or both); cardiac rhythm disturbances; myocardial ischemia; myocardial infarction; cardiac orvascularaneurysm; vasculitis, stroke; peripheral obstructive arteriopathy of a limb, an organ, or a tissue; reperfusion injury following ischemia of the brain, heart or other organ or tissue, endotoxic, surgical, or traumatic shock; hypertension, valvular heart disease, heart failure, abnormal blood pressure; shock; vasoconstriction (including that associated with migraines); vascular abnormality, inflammation and/or insufficiency limited to a single organ or tissue.

In a preferred embodiment, the cardiovascular disease or cardiovascular event which risk is to be detected is selected from the group of fatal- and non-fatal myocardial infarction, stroke, angina pectoris, transient ischemic attacks, peripheral arteriopathy or a combination thereof.

The term "sample", as used herein, refers to any sample from a biological source and includes, without limitation, cell cultures or extracts thereof, biopsied material obtained from a mammal or extracts thereof, and blood, saliva, urine, feces, semen, tears, ar other body fluids or extracts thereof.

When prediction models are used, as far instance, for making treatment decisions, predictive risks are categorized by using risk cutoff thresholds. The term "reclassification", as used herein, refers to the assignation of a person to another category of risk under a new model compared with the initial model of risk assessment. Reclassification is usually referred to as the percentage of persons being reclassified.

The term "Net Reclassification Improvement (NRI)" as used herein, refers to the assessment of the net improvement in risk classification. NRI is calculated as the sum of differences in the proportion of individuals moving up minus the proportion moving down for cases and the proportion of individuals moving down minus the proportion moving up for non-cases. The components of NRI indicate the net benefit of reclassification improvement in cases and non-cases. Positive and negative values represent the net percentage of individuals with improved or worse classification, respectively. Overall, improvement in reclassification is indicated by an NRI of significantly greater than 0.

The term "cardiovascular therapy", as used herein, refers to any type of treatment which results in the amelioration or reduces the risk of suffering any of the above mentioned cardiovascular diseases. Suitable therapies for use in the present invention include, without limitation, anticoagulants, antiplatelet agents, thrombolytic agents, antithrombotics, antiarrhythmic agents, agents that prolong repolarization, antihypertensive agents, vasodilators, antihypertensives, diuretics, inotropic agents, antianginal agents, hypolipemiants or hypolipemic agents and the like.

Non-limiting examples of anticoagulants include acenocoumarol, ancrod, anisindione, bromindione, clorindione, coumetarol, cyclocumarol, dextran sulfate sodium, dicumarol, diphenadione, ethyl biscoumacetate, ethylidene dicoumarol, fluindione, heparin, hirudin, lyapolate sodium, oxazidione, pentosan polysulfate, phenindione, phenprocoumon, phosvitin, picotamide, tioclomarol and warfarin.

Non-limiting examples of antiplatelet agents include aspirin, a dextran, dipyridamole (persantin), heparin, sulfinpyranone (anturane), clopidrogel and ticlopidine (ticlid).

Non-limiting examples of thrombolytic agents include tissue plaminogen activator (activase), plasmin, pro-urokinase, urokinase (abbokinase), streptokinase (streptase), anistreplase/ APSAC (eminase).

Non-limiting examples of antithrombotics include anagrelide, argatroban, cilstazol, daltroban, defibrotide, enoxaparin, fraxiparine, indobufen, lamoparan, ozagrel, picotamide, plafibride, tedelparin, ticlopidine and triflusal. Non-limiting examples of antiarrhythmic agents include Class I antiarrhythmic agents (sodium channel blockers), Class II antiarrhythmic agents (beta -adrenergic blockers), Class III antiarrhythmic agents (repolarization prolonging drugs), Class IV antiarrhythmic agents (calcium channel blockers) and miscellaneous antiarrhythmic agents.

Non-limiting examples of sodium channel blockers include Class IA, Class IB and Class IC antiarrhythmic agents. Non-limiting examples of Class IA antiarrhythmic agents include dispyramide (norpace), procainamide (pronestyl) and quinidine (quinidex). Non- limiting examples of Class IB antiarrhythmic agents include lidocaine (xylocaine), tocainide (tonocard) and mexiletine (mexitil).

Non-limiting examples of Class IC antiarrhythmic agents include encainide (enkaid) and fiecainide (tambocor).

Non-limiting examples of beta blockers, otherwise known as beta-adrenergic blockers, beta-adrenergic antagonists or Class II antiarrhythmic agents, include acebutolol (sectral), alprenolol, amosulalol, arotinolol, atenolol, befunolol, betaxolol, bevantolol, bisoprolol, bopindolol, bucumolol, bufetolol, bufuralol, bunitrolol, bupranolol, butidrine hydrochloride, butofilolol, carazolol, carteolol, carvedilol, celiprolol, cetamolol, cloranolol, dilevalol, epanolol, esmolol (brevibloc), indenolol, labetalol, levobunolol, mepindolol, metipranolol, metoprolol, moprolol, nadolol, nadoxolol, nifenalol, nipradilol, oxprenolol, penbutolol, pindolol, practolol, pronethalol, propanolol (inderal), sotalol (betapace), sulfinalol, talinolol, tertatolol, timolol, toliprolol and xibinolol. In certain embodiments, the beta-blocker comprises an aryloxypropannolamine derivative.

Non-limiting examples of aryloxypropanolamine derivatives include acebutolol, alprenolol, arotinolol, atenolol, betaxolol, bevantolol, bisoprolol, bopindolol, bunitrolol, butofilolol, carazolol, carteolol, carvedilol, celiprolol, cetamolol, epanolol, indenolol, mepindolol, metipranolol, metoprolol, moprolol, nadolol, nipradilol, oxprenolol, penbutolol, pindolol, propanolol, talinolol, tertatolol, timolol and toliprolol.

Non-limiting examples of agents with hypolipemic capabilities include, without limitation, bile acid sequestrants such as quaternary amines (e. g. cholestyramine and colestipol); nicotinic acid and its derivatives; HMG-CoA reductase inhibitors such as mevastatin, pravastatin, and simvastatin; gem fibrozil and other fibric acids, such as clofibrate, fenofibrate, benzafibrate and cipofibrate; probucol; raloxifene and its derivatives.

Non- limiting examples of agents that prolong repolarization, also known as Class III antiarrhythmic agents, include amiodarone (cordarone) and sotalol (betapace).

Non-limiting examples of calcium channel blockers, otherwise known as Class IV antiarrhythmic agent, include an arylalkylamine (e.g., bepridile, diltiazem, fendiline, gallopamil, prenylamine, terodiline, verapamil), a dihydropyridine derivative (felodipine, isradipine, nicardipine, nifedipine, nimodipine, nisoldipine, nitrendipine) a piperazinide derivative (e.g., cinnarizine, flunarizine, lidoflazine) or a micellaneous calcium channel blocker such as bencyclane, etafenone, magnesium, mibefradil or perhexiline. In certain embodiments a calcium channel blocker comprises a long-acting dihydropyridine (nifedipine-type) calcium antagonist.

Non-limiting examples of miscellaneous antiarrhythmic agents include adenosine (adenocard), digoxin (lanoxin), acecainide, ajmaline, amoproxan, aprindine, bretylium tosylate, bunaftine, butobendine, capobenic acid, cifenline, disopyranide, hydro quinidine, indecainide, ipatropium bromide, lidocaine, lorajmine, lorcainide, meobentine, moricizine, pirmenol, prajmaline, propafenone, pyrinoline, quinidine polygalacturonate, quinidine sulfate and viquidil.

Non-limiting examples of antihypertensive agents include sympatholytic, alpha I beta blockers, alpha blockers, anti-angiotensin II agents, beta blockers, calcium channel blockers, vasodilators and miscellaneous antihypertensives.

Non-limiting examples of alpha-blockers, also known as alpha-adrenergic blocker or an alpha-adrenergic antagonist, include amosulalol, arotinolol, dapiprazole, doxazosin, ergoloid mesylates, fenspiride, indoramin, labetalol, nicergoline, prazosin, terazosin, tolazoline, trimazosin and yohimbine. In certain embodiments, an $\alpha$ blocker may comprise a quinazoline derivative.

Non-limiting examples of quinazoline derivatives include alfuzosin, bunazosin, doxazosin, prazosin, terazosin and trimazosin. In certain embodiments, an antihypertensive agent is both an $\alpha$ and beta adrenergic antagonist. Non-limiting examples of an alpha/beta blocker comprise labetalol (normodyne, trandate).

Non-limiting examples of anti-angiotensin II agents include angiotensin converting enzyme inhibitors and angiotensin II receptor antagonists. Non-limiting examples of angiotensin converting enzyme inhibitors (ACE inhibitors) include alacepril, enalapril (vasotec), captopril, cilazapril, delapril, enalaprilat, fosinopril, lisinopril, moveltopril, perindopril, quinapril and ramipril. Non-limiting examples of angiotensin II receptor blocker, also known as angiotensin II receptor antagonist, ANG receptor blockers or an ANG-II type-1 receptor blocker (ARBS), include angiocandesartan, eprosartan, irbesartan, losartan and valsartan. Non-limiting examples of sympatholytics include centrally acting sympatholytics or peripherially acting sympatholytics. Non-limiting examples of centrally acting sympatholytics, also known as central nervous system (CNS) sympatholytics, include clonidine (catapres), guanabenz (wytensin) guanfacine (tenex) and methyldopa (aldomet). Non-limiting examples of a peripherally acting sympatholytic include ganglion blocking agents, an adrenergic neuron blocking agent, a beta-adrenergic blocking agent or an al-adrenergic blocking agent. Non-limiting examples of ganglion blocking agents include mecamylamine (inversine) and trimethaphan (arfonad). Non-limiting examples of adrenergic neuron blocking agents include guanethidine (ismelin) and reserpine (serpasil). Non-limiting examples of beta-adrenergic blockers include acenitolol (sectral), atenolol (tenormin), betaxolol (kerlone), carteolol (cartrol), labetalol (normodyne, trandate), metoprolol (lopressor), nadanol (corgard), penbutolol (levatol), pindolol (visken), propranolol (inderal) and timolol (blocadren). Non-limiting examples of alpha-adrenergic blockers include prazosin (minipress), doxazocin (cardura) and terazosin (hytrin).

In certain embodiments, a cardiovasculator therapeutic agent may comprise a vasodilator (e.g., a cerebral vasodilator, a coronary vasodilator or a peripheral vasodilator). In certain preferred embodiments, a vasodilator comprises a coronary vasodilator. Non-limiting examples of coronary vasodilators include amotriphene, bendazol, benfurodil hemisuccinate, benziodarone, chloracizine, chromonar, clobenfurol, clonitrate, dilazep, dipyridamole, droprenilamine, efloxate, erythrityl tetranitrane, etafenone, fendiline, floredil, ganglefene, herestrol bis(beta - diethylaminoethyl ether), hexobendine, itramin tosylate, khellin, lidoflanine, mannitol hexanitrane, medibazine, nicorglycerin, pentaerythritol tetranitrate, pentrinitrol, perhexiline, pimefylline, trapidil, tricromyl, trimetazidine, trolnitrate phosphate and visnadine.

In certain embodiments, a vasodilator may comprise a chronic therapy vasodilator or a hypertensive emergency vasodilator. Non-limiting examples of a chronic therapy vasodilator include hydralazine (apresoline) and minoxidil (loniten). Non-limiting examples of a hypertensive emergency vasodilator include nitroprusside (nipride), diazoxide (hyperstat IV), hydralazine (apresoline), minoxidil (loniten) and verapamil.

Non-limiting examples of miscellaneous antihypertensives include ajmaline, gamma-aminobutyric acid, bufeniode, cicletainine, ciclosidomine, a cryptenamine tannate, fenoldopam, flosequinan, ketanserin, mebutamate, mecamylamine, methyldopa, methyl 4-pyridyl ketone thiosemicarbazone, muzolimine, pargyline, pempidine, pinacidil, piperoxan, primaperone, a protoveratrine, raubasine, rescimetol, rilmenidene, saralasin, sodium nitrorusside, ticrynafen, trimethaphan camsylate, tyrosinase and urapidil.

In certain embodiments, an antihypertensive may comprise an arylethanolamine derivative, a benzothiadiazine derivative, a 7N-carboxyalkyl(peptide/lactam) derivative, a dihydropyridine derivative, a guanidine derivative, a hydrazines/phthalazine, an imidazole derivative, a quaternary ammonium compound, a reserpine derivative or a suflonamide derivative. Non-limiting examples of arylethanolamine derivatives include amosulalol, bufuralol, dilevalol, labetalol, pronethalol, sotalol and sulfinalol. Non-limiting examples of benzothiadiazine derivatives include althizide, bendroflumethiazide, benzthiazide, benzylhydrochlorothiazide, buthiazide, chlorothiazide, chlorthalidone, cyclopenthiazide, cyclothiazide, diazoxide, epithiazide, ethiazide, fenquizone, hydrochlorothizide, hydroflumethizide, methyclothiazide, meticrane, metolazone, paraflutizide, polythizide, tetrachlormethiazide and trichlormethiazide. Non-limiting examples of N-carboxyalkyl (peptide/lactam) derivatives include alacepril, captopril, cilazapril, delapril, enalapril, enalaprilat, fosinopril, lisinopril, moveltipril, perindopril, quinapril and ramipril. Non-limiting examples of dihydropyridine derivatives include amlodipine, felodipine, isradipine, nicardipine, nifedipine, nilvadipine, nisoldipine and nitrendipine. Non-limiting examples of guanidine derivatives include bethanidine, debrisoquin, guanabenz, guanacline, guanadrel, guanazodine, guanethidine, guanfacine, guanochlor, guanoxabenz and guanoxan. Non-limiting examples of hydrazines/phthalazines include budralazine, cadralazine, dihydralazine, endralazine, hydracarbazine, hydralazine, pheniprazine, pildralazine and todralazine. Non-limiting examples of imidazole derivatives include clonidine, lofexidine, phentolamine, tiamenidine and tolonidine. Non-limiting examples of quaternary ammonium compounds include azamethonium bromide, chlorisondamine chloride, hexamethonium, pentacynium bis(methylsulfate), pentamethonium bromide, pentolinium tartrate, phenactropinium chloride and trimethidinium methosulfate. Non-limiting examples of reserpine derivatives include bietaserpine, deserpidine, rescinnamine, reserpine and syrosingopine. Non-limiting examples of sulfonamide derivatives include ambuside, clopamide, furosemide, indapamide, quinethazone, tripamide and xipamide. Vasopressors generally are used to increase blood pressure during shock, which may occur during a surgical procedure. Non-limiting examples of a vasopressor, also known as an antihypotensive, include amezinium methyl sulfate, angiotensin amide, dimetofrine, dopamine, etifelmin, etilefrin, gepefrine, metaraminol, midodrine, norepinephrine, pholedrine and synephrine. Non-limiting examples of agents for the treatment of congestive heart failure include anti-angiotensin II agents, afterload-preload reduction treatment, diuretics and inotropic agents.

In certain embodiments, an animal, e.g. a human, patient that cannot tolerate an angiotensin antagonist may be treated with a combination therapy. Such therapy may combine administration of hydralazine (apresoline) and isosorbide dinitrate (isordil, sorbitrate).

Non-limiting examples of diuretics include a thiazide or benzothiadiazine derivative (e.g., althiazide, bendroflumethazide, benzthiazide, benzylhydrochlorothiazide, buthiazide, chlorothiazide, chlorothiazide, chlorthalidone, cyclopenthiazide, epithiazide, ethiazide, ethiazide, fenquizone, hydrochlorothiazide, hydroflumethiazide, methyclothiazide, meticrane, metolazone, paraflutizide, polythizide, tetrachloromethiazide, trichlormethiazide), an organomercurial (e.g., chlormerodrin, meralluride, mercamphamide, mercaptomerin sodium, mercumallylic acid, mercumatilin dodium, mercurous chloride, mersalyl), a pteridine (e.g., furterene, triamterene), purines (e.g., acefylline , 7-morpholinomethyltheophylline, pamobrom, protheobromine, theobromine), steroids including aldosterone antagonists (e.g., canrenone, oleandrin, spironolactone), a sulfonamide derivative (e.g., acetazolamide, ambuside, azosemide, bumetanide, butazolamide, chloraminophenamide, clofenamide, clopamide, clorexolone, diphenylmethane-4,4'-disulfonamide, disulfamide, ethoxzolamide, furosemide, indapamide, mefruside, methazolamide, piretanide, quinethazone, torasemide, tripamide, xipamide), an uracil (e.g., aminometradine, amisometradine), a potassium sparing antagonist (e.g., amiloride, triamterene) or a miscellaneous diuretic such as aminozine, arbutin, chlorazanil, ethacrynic acid, etozolin, hydracarbazine, isosorbide, mannitol, metochalcone, muzolimine, perhexiline, ticrnafen and urea. Non-limiting examples of positive inotropic agents, also known as cardiotonics, inelude acefylline, an acetyldigitoxin, 2-amino-4-picoline, amrinone, benfurodil hemisuccinate, bucladesine, cerberosine, camphotamide, convallatoxin, cymarin, denopamine, deslanoside, digitalin, digitalis, digitoxin, digoxin, dobutamine, dopamine, dopexamine, enoximane, erythrophleine, fenalcomine, gitalin, gitoxin, glycocyamine, heptaminol, hydrastinine, ibopamine, a lanatoside, metamivam, milrinone, nerifolin, oleandrin, ouabain, oxyfedrine, prenalterol, proscillaridine, resibufogenin, scillaren, scillarenin, strphanthin, sulmazole, theobromine and xamoterol.

In particular embodiments, an intropic agent is a cardiac glycoside, beta-adrenergic agonist or a phosphodiesterase inhibitor. Non-limiting examples of cardiac glycosides include digoxin (lanoxin) and digitoxin (crystodigin). Non-limiting examples of beta - adrenergic agonists include albuterol, bambuterol, bitolterol, carbuterol, clenbuterol, clorprenaline, denopamine, dioxethedrine, dobutamine (dobutrex), dopamine (intropin), dopexamine, ephedrine, etafedrine, ethy norepinephrine, fenoterol, formoterol, hexoprenaline, ibopamine, isoetharine, isoproterenol, mabuterol, metaproterenol, methoxyphenamine, oxyfedrine, pirbuterol, procaterol, protokylol, reproterol, rimiterol, ritodrine, soterenol, terbutaline, tretoquinol, tulobuterol and xamoterol. Non-limiting examples of a phosphodiesterase inhibitor include amrinone (inocor).

Antianginal agents may comprise organonitrates, calcium channel blockers, beta blockers and combinations thereof. Non-limiting examples of organonitrates, also known as nitrovasodilators, include nitroglycerin (nitro-bid, nitrostat), isosorbide dinitrate (isordil, sorbitrate) and amyl nitrate (aspirol, vaporole). Endothelin (ET) is a 21 - amino acid peptide that has potent physiologic and pathophysiologic effects that appear to be involved in the development of heart failure. The effects of ET are mediated through interaction with two classes of cell surface receptors. The type A receptor (ET-A) is associated with vasoconstriction and cell growth while the type B receptor (ET-8) is associated with endothelial-cell mediated vasodilation and with the release of other neurohormones, such as aldosterone. Pharmacologic agents that can inhibit either the production of ET or its ability to stimulate relevant cells are known in the art. Inhibiting the production of ET involves the use of agents that block an enzyme termed endothelin-converting enzyme that is involved in the processing of the active peptide from its precursor. Inhibiting the ability of ET to stimulate cells involves the use of agents that block the interaction of ET with its receptors. Non-limiting examples of endothelin receptor antagonists (ERA) include Bosentan, Enrasentan, Ambrisentan, Darusentan, Tezosentan, Atrasentan, Avosentan, Clazosentan, Edonentan, sitaxsentan, TBC 3711, BQ 123, and BQ 788.

Those skilled in the art will readily recognize that the analysis of the nucleotides present according to the method of the invention in an individual's nucleic acid can be done by any method or technique capable of determining nucleotides present in a polymorphic site. As it is obvious in the art, the nucleotides present in the polymorphic markers can be determined from either nucleic acid strand or from both strands.

Once a biological sample from a subject has been obtained (e.g., a bodily fluid, such as urine, saliva, plasma, serum, or a tissue sample, such as a buccal tissue sample or a buccal cell), detection of a sequence variation or allelic variant SNP is typically undertaken. Virtually any method known to the skilled artisan can be employed. Perhaps the most direct method is to actually determine the sequence of either genomic DNA or cDNA and compare these sequences to the known alleles SNPs of the gene. This can be a fairly expensive and time-consuming process. Nevertheless, this technology is quite common and is well known.

Any of a variety of methods that exist for detecting sequence variations may be used in the methods of the invention. The particular method used is not important in the estimation of cardiovascular risk or treatment selection.

Other possible commercially available methods exist for high throughput SNP identification not using direct sequencing technologies. For example, Illumina's Veracode Technology, Taqman® SNP Genotyping Chemistry and KASPar SNP genotyping Chemistry.

A variation on the direct sequence determination method is the Gene Chip(™) method available from Affymetrix. Alternatively, robust and less expensive ways of detecting DNA sequence variation are also commercially available. For example, Perkin Elmer adapted its TAQman Assay(™) to detect sequence variation. Orchid BioSciences has a method called SNP-IT (™) (SNP-Identification Technology) that uses primer extension with labeled nucleotide analogs to determine which nucleotide occurs at the position immediately 3' of an oligonucleotide probe, the extended base is then identified using direct fluorescence, an indirect colorimetric assay, mass spectrometry, or fluorescence polarization. Sequenom uses a hybridization capture technology plus MALDI-TOF (Matrix Assisted Laser Desorption/Ionization--Time-of-Flight mass spectrometry) to detect SNP genotypes with their MassARRAY(™) system. Promega provides the READIT(™) SNP/Genotyping System (U.S. Pat. No. 6,159,693). In this method, DNA or RNA probes are hybridized to target nucleic acid sequences. Probes that are complementary to the target sequence at each base are depolymerized with a proprietary mixture of enzymes, while probes which differ from the target at the interrogation position remain intact. The method uses pyrophosphorylation chemistry in combination with luciferase detection to provide a highly sensitive and adaptable SNP scoring system. Third Wave Technologies has the Invader OS(™) method that uses proprietary Cleavaseg enzymes, which recognize and cut only the specific structure formed during the Invader process. Invader OS relies on linear amplification of the signal generated by the Invader process, rather than on exponential amplification of the target. The Invader OS assay does not utilize PCR in any part of the assay. In addition, there are a number of forensic DNA testing labs and many research labs that use gene-specific PCR, followed by restriction endonuclease digestion and gel electrophoresis (or other size separation technology) to detect restriction fragment length polymorphisms (RFLPs).

In various embodiments of any of the above aspects, the presence or absence of the SNPs is identified by amplifying or failing to amplify an amplification product from the sample. Polynucleotide amplifications are typically template-dependent. Such amplifications generally rely on the existence of a template strand to make additional copies of the template. Primers are short nucleic acids that are capable of priming the synthesis of a nascent nucleic acid in a template-dependent process, which hybridize to the template strand. Typically, primers are from ten to thirty base pairs in length, but longer sequences can be employed. Primers may be provided in double-stranded and/or single-stranded form, although the single-stranded form generally is preferred. Often, pairs of primers are designed to selectively hybridize to distinct regions of a template nucleic acid, and are contacted with the template DNA under conditions that permit selective hybridization. Depending upon the desired application, high stringency hybridization conditions may be selected that will only allow hybridization to sequences that are completely complementary to the primers. In other embodiments, hybridization may occur under reduced stringency to allow for amplification of nucleic acids containing one or more mismatches with the primer sequences. Once hybridized, the template-primer complex is contacted with one or more enzymes that facilitate template-dependent nucleic acid synthesis. Multiple rounds of amplification, also referred to as "cycles," are conducted until a sufficient amount of amplification product is produced.

Polymerase Chain Reaction

A number of template dependent processes are available to amplify the oligonucleotide sequences present in a given template sample. One of the best known amplification methods is the polymerase chain reaction. In PCR, pairs of primers that selectively hybridize to nucleic acids are used under conditions that permit selective hybridization. The term "primer", as used herein, encompasses any nucleic acid that is capable of priming the synthesis of a nascent nucleic acid in a template-dependent process. Primers may be provided in double-stranded or single-stranded form, although the single-stranded form is preferred. Primers are used in any one of a number of template dependent processes to amplify the target gene sequences present in a given template sample. One of the best known amplification methods is PCR, which is described in detail in U.S. Pat. Nos. 4,683,195; 4,683,202 and 4,800,159. In PCR, two primer sequences are prepared which are complementary to regions on opposite complementary strands of the target-gene(s) sequence. The primers will hybridize to form a nucleic-acid:primer complex if the target- gene(s) sequence is present in a sample. An excess of deoxyribonucleoside triphosphates is added to a reaction mixture along with a DNA polymerase, e.g. Taq polymerase, that facilitates template-dependent nucleic acid synthesis. If the target-gene(s) sequence primer complex has been formed, the polymerase will cause the primers to be extended along the target-gene(s) sequence by adding on nucleotides. By raising and lowering the temperature of the reaction mixture, the extended primers will dissociate from the target-gene(s) to form reaction products, excess primers will bind to the target-gene(s) and to the reaction products and the process is repeated. These multiple rounds of amplification, referred to as "cycles", are conducted until a sufficient amount of amplification product is produced. The amplification product may be digested with a restriction enzyme before analysis. In still other embodiments of any of the above aspects, the presence or absence of the SNP is identified by hybridizing the nucleic acid sample with a primer labeled with a detectable moiety. In other embodiments of any of the above aspects, the detectable moiety is detected in an enzymatic assay,, radioassay, immunoassay, or by detecting fluorescence. In other embodiments of any of the above aspects, the primer is labeled with a detectable dye (e.g., SYBR Green 1, YO-PR0-1, thiazole orange, Hex, pico green, edans, fluorescein, FAM, or TET). Non-fluorescent chromophores may also be used, e.g. quenchers, e.g. dark quenchers, e.g. Black Hole Quencher-1 (BHQ-1). In other embodiments of any of the above aspects, the primers are located on a chip. In other embodiments of any of the above aspects, the primers for amplification are specific for said SNPs.

For example, in some embodiments, probes disclosed herein may be labeled for use in fluorescence-based methods as mentioned above at one end (e.g. the 5' terminus) with a dye (e.g. a fluorescent daye; e.g. FAM or Hex), and at the other end (e.g. the 3' terminus) with a quencher, e.g. with BHQ-1. E.g., for SEQ ID NOs:58 and 59, respectively, such an approach may result in molecules represented as FAM-AATGGAAGTATcATACACTGCTGATGG-BHQ1 and HEX-AAATGGAAGTATtATACACTGCTGATGG-BHQ1. E.g., for SEQ ID NOs:64 and 65, respectively, such an approach may result in molecules represented as
FAM-AGGATTGAGcGAGTCAGGC-BHQ1 and
HEX-TAGGATTGAGtGAGTCAGGC-BHQ1.

Another method for amplification is the ligase chain reaction ("LCR"). LCR differs from PCR because it amplifies the probe molecule rather than producing an amplicon through polymerization of nucleotides. In LCR, two complementary probe pairs are prepared, and in the presence of a target sequence, each pair will bind to opposite complementary strands of the target such that they abut. In the presence of a ligase, the two probe pairs will link to form a single unit. By temperature cycling, as in PCR, bound ligated units dissociate from the target and then serve as "target sequences" for ligation of excess probe pairs. U.S. Pat. No. 4,883,750, incorporated herein by reference, describes a method similar to LCR for binding probe pairs to a target sequence.

Isothermal Amplification

An isothermal amplification method, in which restriction endonucleases and ligases are used to achieve the amplification of target molecules that contain nucleotide 5'-[[alpha]-thio]-triphosphates in one strand of a restriction site also may be useful in the amplification of nucleic acids in the present invention. In one embodiment, loop-mediated isothermal amplification (LAMP) method is used for single nucleotide polymorphism (SNP) typing.

Strand Displacement Amplification

Strand Displacement Amplification (SDA) is another method of carrying out isothermal amplification of nucleic acids which involves multiple rounds of strand displacement and synthesis, i.e., nick translation. A similar method, callad Repair Chain Reaction (RCR), involves annealing several probes throughout a region targeted for amplification, followed by a repair reaction in which only two of the four bases are present. The other two bases can be added as biotinylated derivatives for easy detection.

Transcription-Based Amplification

Other nucleic acid amplification procedures include transcription-based amplification systems, including nucleic acid sequence based amplification. In nucleic acid sequence based amplification, the nucleic acids are prepared for amplification by standard phenol/chloroform extraction, heat denaturation of a clinical sample, treatment with lysis buffer and minispin columns for isolation of DNA and RNA or guanidinium chloride extraction of RNA. These amplification techniques involve annealing a primer, which has large specific sequences. Following polymerization, DNA/RNA hybrids are digested with RNase H while double stranded DNA molecules are heat denatured again. In either case the single stranded DNA is made fully double stranded by addition of second target specific primer, followed by polymerization. The double-stranded DNA molecules are then multiply transcribed by a polymerase such as T7 or SP6. In an isothermal cyclic reaction, the RNA's are reverse transcribed into double stranded DNA, and transcribed once against with a polymerase such as T7 or SP6. The resulting products, whether truncated or complete, indicate target specific sequences. Other amplification methods may be used in accordance with the present invention. In one embodiment, "modified" primers are used in a PCR-like, template and enzyme dependent synthesis. The primers may be modified by labeling with a capture moiety (e.g., biotin) and/or a detector moiety (e.g., enzyme). In the presence of a target sequence, the probe binds and is cleaved catalytically. After cleavage, the target sequence is released intact to be bound by excess probe. Cleavage of the labeled probe signals the presence of the target sequence. In another approach, a nucleic acid amplification process involves cyclically synthesizing single-stranded RNA ("ssRNA"), ssDNA, and double-stranded DNA (dsDNA), which may be used in accordance with the present invention. The ssRNA is a first template for a first primer oligonucleotide, which is elongated by reverse transcriptase (RNA-dependent DNA polymerase). The RNA is then removed from the resulting DNA:RNA duplex by the action of ribonuclease H (RNase H, an RNase specific for RNA in duplex with either DNA or RNA). The resultant ssDNA is a second template for a second primer, which also includes the sequences of an RNA polymerase promoter (exemplified by T7 RNA polymerase) 5' to its homology to the template. This primer is then extended by DNA polymerase (exemplified by the large "Klenow" fragment of E. co/i DNA polymerase 1), resulting in a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

Methods for Nucleic Acid Separation

It may be desirable to separate nucleic acid products from other materials, such as template and excess primer. In one embodiment, amplification products are separated by agarose, agarose-acrylamide or polyacrylamide gel electrophoresis using standard methods (Sambrook et al., 1989, see infra). Separated amplification products may be cutout and eluted from the gel for further manipulation. Using low melting point agarose gels, the separated band may be removed by heating the gel, followed by extraction of the nucleic acid. Separation of nucleic acids may also be effected by chromatographic techniques known in the art. There are many kinds of chromatography which may be used in the practice of the present invention, including adsorption, partition, ionexchange, hydroxylapatite, molecular sieve, reverse-phase, column, paper, thin-layer, and gas chromatography as well as HPLC. In certain embodiments, the amplification products are visualized. A typical visualization method involves staining of a gel with ethidium bromide and visualization of bands under UV light. Alternatively, if the amplification products are integrally labeled with radio- or fluorometrically-labeled nucleotides, the separated amplification products can be exposed to X-ray film or visualized with light exhibiting the appropriate excitatory spectra.

Alternatively, the presence of the polymorphic positions according to the methods of the invention can be determined by hybridisation or lack of hybridisation with a suitable nucleic acid probe specific for a polymorphic nucleic acid but not with the non-mutated nucleic acid. By "hybridize" is meant a pair to form a double-stranded molecule between complementary polynucleotide sequences, or portions thereof, under various conditions of stringency. For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30°C, more preferably of at least about 37°C, and most preferably of at least about 42°C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred embodiment, hybridization will occur at 30°C in 750 mM NaCl, 75 mM trisodium citrate, and 1% SDS. In a more preferred embodiment, hybridization will occur at 37°C in 500 mM NaCl, 50 mM trisodium citrate, 1% SDS, 35% formamide, and 100 [μ]g/ml denatured salman sperm DNA (ssDNA). In a most preferred embodiment, hybridization will occur at 42°C in 250 mM NaCl, 25 mM trisodium citrate, 1% SDS, 50% formamide, and 200 [μ]g/ml ssDNA. Useful variations on these conditions will be readily apparent to those skilled in the art. For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25°C, more preferably of at least about 42°C, and even more preferably of at least about 68°C. In a preferred embodiment, wash steps will occur at 25°C in 30 mM NaCl, 3 mM trisodium citrate, and 0.1% SDS. In a more preferred embodiment, wash steps will occur at 42°C in 15 mM NaCl, 1.5 mM trisodium citrate, and 0.1% SDS. In a more preferred embodiment, wash steps will occur at 68°C in 15 mM NaCl, 1.5 mM trisodium citrate, and 0.1% SDS. Additional variations on these conditions will be readily apparent to those skilled in the art. Hybridization techniques are well known to those skilled in the art and are described, for example, in Benton and Oavis (Science 196: 180, 1977); Grunstein and Hogness (Proc, Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York, 1989.

Nucleic acid molecules useful for hybridisation in the methods of the invention include any nucleic acid molecule which exhibits substantial identity so as to be able to specifically hybridise with the target nucleic acids. Polynucleotides having "substantial identity" to an endogenous sequence are typically capable of hybridizing with at least one strand of a double-stranded nucleic acid molecule. By "substantially identical" is meant a polypeptide or nucleic acid molecule exhibiting at least 50% identity to a reference amino acid sequence or nucleic acid sequence. Preferably, such a sequence is at least 60%, more preferably 80% or 85%, and more preferably 90%, 95% or even 99% identical at the amino acid level or nucleic acid to the sequence used for comparison. Sequence identity is typically measured using sequence analysis software (for example, Sequence Analysis Software Package of the Genetics Computer Group, University of Wisconsin Biotechnology Center, 1710 University Avenue , Madison, Wis. 53705, BLAST, BESTFIT, GAP, or PILEUP/PRETTYBOX programs). Such software matches identical ar similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between e<"3> and e<"100> indicating a closely related sequence.

A detection system may be used to measure the absence, presence, and amount of hybridization for all of the distinct sequences simultaneously. Preferably, a scanner is used to determine the levels and patterns of fluorescence.

<u>Method to reclassify the patients to a more appropriate risk status.</u>

Another object of the present invention is the improvement of the cardiovascular risk scales/methods/functions nowadays in use by the particular combination of clinic/biochemical data and SNP markers as set out in table 1 SEQ ID No. 1-11 of Table 1, as explained above, associated with a risk of cardiovascular disease/disorder and/or with a risk of cardiovascular disease complications or event including, but not limited to, myocardial infarction, stroke, angina pectoris, transient ischemic attacks, congestive heart failure, aortic aneurysm and death. Cardiovascular risk factors nowadays in use include, but are not limited to, the original Framingham function, the adapted Framingham function (such as but not limited to REGICOR function), PROCAM function, SCORE function, QRISK function, and the MACE and total mortality risk calculator. The improvement of the Framingham, PROCAM, REGICOR, QRISK, and MACE and total mortality risk calculator functions is obtained when SNP markers shown in table 1 and new clinical/biochemical data corresponding to the variables listed in table C are included into the original equations.

For example, the equation 2a

$$w_i = \beta_{chol} * (cholesterol_i - 6) + \beta_{SBP} * (SBP_i - 120) + \beta_{smoker} * current_i$$

is transformed to equation 2b

$$w_i = \beta_{chol} * (cholesterol_i - 6) + \beta_{SBP} * (SBP_i - 120) + \beta_{smoker} * current_i + \beta n * (n_i - \overline{n}) + \beta_{GRS_i} * (GRS_i - GRS)$$

The cardiovascular risk will be calculated using the equation 2b using the SCORE risk function following several steps:

First step: compute linear combination of risk factors:

$$w_i = \beta_{chol} * (cholesterol_i - 6) + \beta_{SBP} * (SBP_i - 120) + \beta_{smoker} * current_i + \beta n_{-} * (n_i - n) + \overline{\beta}_{GRS_i} * (GRS_i - \overline{GRS})$$

where

$\beta_{chol}$: logarithm of hazard ratio corresponding to the cholesterol (Table D),
cholesterol,: cholesterol level for the individual "i" in mmol/L,
$\beta_{SBP}$: logarithm of hazard ratio corresponding to systolic blood pressure (Table D),
$SBR_i$: systolic blood pressure for the individual "i" in mm Hg,
$\beta_{smoker}$: logarithm of hazard ratio corresponding to being a smoker (Table D),
$current_i$: current smoking status for the individual "i" (1:current, 0:former/never),
$\beta_{n_i}$: is the logarithm of the hazard ratio corresponding to the clinical/biochemical variable "n" from the data shown in table A in the subject "i".
$n_i$: the value of each for an individual "i",
$\overline{n}$ : is the mean value of the clinical/biochemical variable "n" in the population
$\beta_{GRS}$: logarithm of the hazard ratio corresponding to one unit increase in the value of the genetic risk score. The value of this $\beta_{GRS}$ is 0.140 with a range of values going from 0.010 to 0.500,
$GRS_i$: genetic risk for the individual "i" defined as the weighted number of risk alleles (0, 1, 2) for the genetic variants included in the equation for an individual "i". The variants currently included in the genetic risk score are shown in table A. The weights are proportional to the betas of each SNP included in the score (shown in table B), and the range of GRS goes from 0 to twice the number of SNPs included in the score,
GRS: means the value of the genetic risk score in the population,

Second step: compute baseline survival.

$$S_0(age) = \exp\{-\exp(\alpha) * (age - 20)^p\}$$

$$S_0(age + 10) = \exp\{-\exp(\alpha) * (age - 10)^p\}$$

where

$\alpha$, p: shape and scale parameters of the weibull distribution. Their values are shown in Table E (parameters)
exp: natural exponentiation

Third step: compute 10 years survival

$$S(age) = \{S_0(age)\}^{\exp(w')}$$

$$S(age+10) = \{S_0(age+10)\}^{\exp(w')}$$

$$S_{10}(age) = S(age+10) / S(age)$$

Fourth step: compute probability of having the event during the 10 years follow-up

$$Risk_{10}(age) = 1 - S_{10}(age)$$

Fifth step: compute the probability of having a cardiovascular event during the 10 years follow-up as the sum of coronary and non-coronary cardiovascular risk.

$$CVDRisk_{10} = [CHDRisk_{10}(age)] + [Non - CHDRisk_{10}(age)]$$

Table A. List of coronary risk factors included in the present inventive model, logarithm of hazard ratio, $\beta_{CRFp}$ for every clinical/biochemical coronary risk factor, $CRF_p$ range of the average values for the cinical/biochemical coronary risk factor "p" in the population. One or more of these clinical/biochemical risk factors can be selected for risk estimation.

| $CRF_p$ | $\beta_{CRFp}$ | $\overline{CRF}_p$ |
|---|---|---|
| Age (10 years) | 0.086 (0.008;0.860) | 15-85 |
| Sex (male) | 0.668 (0.067; 1.500) | 0.1 |
| Total cholesterol (mg/dL) | 0 (-0.100;0-100) | 80 to 600 |
| LDL-cholesterol | 0 (-0.100;0-100) | 45 to 500 |
| HDL-cholesterol (mg/dL) | -0.223 (-0.600;-0.020) | 20 to 120 |
| Triglyceride (mg/dL) | 0 (-0.100;0-100) | 40 to 500 |
| Dyslipemia history (0=N0, 1=Yes) | 0.378 (0.030; 1.200 | 0, 1 |
| Systolic blood pressure (mmHg) | 0 (-0.100;0-200) | 60 to 250 |
| Diastolic blood pressure (mmHg) | 0 (-0.100;0-200) | 40 to 150 |
| Hypertension history (0=No, 1=Yes) | 0 (-0.100;0-300) | 0, 1 |
| Glycaemia (mg/dL) | 0 (-0.100;0-300) | 30 to 350 |
| Glycated hemoglobin (%) | 0 (-0.100;0-300) | 3 to 20 |

(continued)

| $CRF_p$ | $\beta_{CRFp}$ | $\overline{CRF}_p$ |
|---|---|---|
| Diabetes history (0=No, 1=Yes) | 0 (-0.100;0-300) | 0, 1 |
| Renal insufficiency (0=No, 1=Yes) | 0 | 0, 1 |
| Stage Chronic kidney - disease:<br>• Predialysis<br>• Dialysis<br>• Transplantation<br>• Transplantation failure | <br>-0.494 (-2.800; -0.050)<br>-0.654 (-2.000; -0.060)<br>-0.416 (-2.000;-0.040)<br>-0.654 (-2.000;-0.060) | <br>0, 1<br>0, 1<br>0, 1<br>0, 1 |
| Glomerular filtration (mL/min/$m^2$) | 0 (-0.800;0.100) | 1 to 200 |
| Creatinine /mg/dL) | 0 (-0.200;0.900) | 0 to 20 |
| Calcium (mg/dL) | 0 (-0.400;1.500) | 3 to 50 |
| Phosphorus (mg/dL) | 0 (-0.400;1.500) | 0.5 to 15 |
| Parathormone (pg/gL) | 0(-0.400; 1.500) | 0 to 4000 |
| Albumin (g/L) | -0.598(-1.500;-0.060) | 1 to 7 |
| 24 hours proteinuria (mg/24h) | 0(-0.400; 1.500) | 0 to 70 |
| GRS (standard deviation of the GRS)<br>Predialysis<br>Dialysis<br>Transplantation<br>Transplantation failure | <br>0.815 (0.080;2.000)<br>-0.248 (-0.400; 1.000)<br>0.554 (0.050;2.000)<br>0.020 (-0.200;1.000) | <br>-6 to +6<br>-6 to +6<br>-6 to +6<br>-6 to +6 |

Table B. Variants currently included in the present inventive model logarithm of hazard ratio (range of possible values), $^{\beta}SNP_j$ and average risk allele number of copies (range of possible values), $SNP_j$ for every genetic variant.

| $SNP_j$ | $^{\beta}SNP_j$ | $\overline{SNP}_j$ |
|---|---|---|
| rs17465637 | 0.1310 (0.0100-0.5000) | 1.48 (0.3-2.8) |
| rs6725887 | 0.1310 (0.0100-0.5000) | 0.3 (0.15-1.4) |
| rs9818870 | 0.1133 (0.0100-0.5000) | 0.36 (0.15-1-4) |
| rs12526453 | 0.0953 (0.100-0.5000) | 1.34 (0.3-2.8) |
| rs1333049 | 0.2546 (0.0100-1.0000) | 0.92 (0.3-2.8) |
| rs9982601 | 0.1655 (0.0100-0.5000) | 0.3 (0.15-1.4) |
| rs10455872 | 0.2852 (0.0100-1.0000) | 0.14 (0.1-1.4) |
| rs10507391* | 0.1310 (0.0100-0.5000) | 0.08 (0.005-0.5) |
| rs9315051* | 0.0000 (0.00100-0.5000) | 1 (0.3-2.8) |
| rs17222842* | 0.1310 (0.0100-0.5000) | 0.08 (0.05-0.5) |
| rs501120 | 0.090 (0.010; 0.400) | 0.08 (0.05;0.5). |
| *All these SNPs define ALOX5AP haplotype B that has an effect size defined by the β value of 0.1310 | | |

Table C

| | |
|---|---|
| Age | |
| Sex | |

(continued)

| |
|---|
| Total cholesterol (mg/dL) |
| LDL-cholesterol (mg/dL) |
| HDL-cholesterol (mg/dL) |
| Triglyceride (mg/dL) |
| Diagnosis of Dyslipemia (No/Yes) |
| Systolic blood pressure (mmHg) |
| Diastolic blood pressure (mmHg) |
| Diagnosis of Hypertension (No/Yes) |
| Glycaemia (mg/dL) |
| Glycated hemoglobin (%) |
| Diagnosis of Diabetes Mellitus (No/Yes) |
| Renal insufficiency (No/Yes) |
| Stage Chronic kidney disease:<br>Predialysis<br>Dialysis<br>Transplantation<br>Transplantation failure |
| Glomerular filtration (mL/min/$m^2$) |
| Creatinine (mg/dL) |
| Calcium (mg/dL) |
| Phosphorus (mg/dL) |
| Parathormone (pg/gL) |
| Albumin (g/L) |
| 24 hours proteinuria (mg/24h) |

Table D

| | CHD | Non-CHD CVD |
|---|---|---|
| Current smoker, $\beta_{smoker}$ | 0.71 | 0.63 |
| Cholesterol (nmol/L), $\beta_{chol}$ | 0.24 | 0.02 |
| Systolic blood pressure (mmHg), $\beta_{SBP}$ | 0.018 | 0.022 |
| CHD= coronary heart disease | | |
| CVD= cardiovascular disease | | |

Table E

| | | CHD | | | Non-CHD CVD | |
|---|---|---|---|---|---|---|
| Country | | A | p | | A | p |
| Low risk | Men | -22.1 | 4.71 | | -26.7 | 5.64 |
| | Women | -29.8 | 6.36 | | -31.0 | 6.62 |
| High risk | Men | -21.0 | 4.62 | | -25.7 | 5.47 |

(continued)

| | | CHD | | | Non-CHD CVD | |
|---|---|---|---|---|---|---|
| Country | | A | p | | A | p |
| | Women | -28.7 | 6.23 | | -30.0 | 6.42 |
| CHD: coronary heart disease<br>CVD: cardiovascular disease | | | | | | |

Surprisingly, the combination of SNP markers included in the present invention and set forth in table 1 (figure 1) and those combinations included in the different embodiments and using the functions described in functions 1a to 1b above have proved in patients suffering from chronic kidney disease to be capable to estimate the risk to suffer cardiovascular disease and/or cardiovascular events and/or cardiovascular complications with a higher accuracy than that obtained using the classical risk factors alone or using the scales/functions nowadays in use. Surprisingly, the combination of SNP markers included in the present invention and set forth in table 1 (Figure 1) and those combinations included in the different embodiments and using the functions described in functions 1a to 1b above have proved in patients suffering from chronic kidney disease to obtain a higher validity and a superior validation in predicting cardiovascular disease and/or cardiovascular events and/or cardiovascular disease complications than that obtained using the classical risk factors alone and/or using the functions nowadays in use or published functions including genetic information. Moreover, the reclassification was also improved.

By the use of the functions described, a personalized risk is obtained in patients suffering from chronic kidney disease for the development of coronary heart disease and/or cardiovascular events and/or cardiovascular disease, in particular fatal- and non-fatal-myocardium infarction, angina, stroke, transient ischemic attack, peripheral arteriopathy or a combination thereof. The method provided will upgrade (reclassify) those subjects wrongly classified with the methods used nowadays to calculate the cardiovascular risk to a more accurate risk stratum. As the treatment (preventive and/or therapeutic) is adapted to the level of risk, the reclassification will imply the use in a more effective manner the preventive and/or therapeutic measures that will decrease the incidence and/or recurrence of cardiovascular disease and cardiovascular disease complications such as - but not limited to - fatal- and non-fatal-myocardial infarction, angina, stroke, transient ischemic attack, peripheral arteriopathy or a combination thereof.

**Example 1**

Chronic kidney disease (CKD) is an increasingly noted worldwide public health problem associated with increased morbidity and mortality, and cardiovascular disease is a major cause even in pre-ESRD CKD patients (N Eng J Med 2004; 351: 1296).

This excessive cardiovascular risk can be attributed both to the high prevalence of traditional risk factors among CKD patients and to the presence of other non-traditional risk factors (Kidney Int 2006; 70: 26).

Classical coronary heart disease (CHD) risk equations such as the Framingham Risk Score have shown limited clinical utility in CKD patients (J Am Coll Cardiol 2007; 50: 217).

Whether the addition of a genetic risk score (GRS) including genetic variants associated with CHD but not with classical cardiovascular risk factors can improves CHD risk prediction in CKD patients remains unclear.

The objectives of this study are:

1. To develop and assess the predictive capacity of a CHD risk function based on clinical variables
2. To evaluate whether the inclusion of genetic variants improve CHD risk estimation in CKD patients including kidney transplant recipients

Methods:

- Our cohort study included 632 KD patients (200 kidney transplant recipients) with a median follow-up of 9.13 years. Mean age was 53.4 (±10.6) years and 32.4% of the patients were male.
- Seventy three coronary events (fatal- or non-fatal acute myocardial infarct or acute angina) were identified in the follow-up.
- The clinical CHD risk score included:

- KD status (CKD stages 1-4, ESRD in renal replacement therapy or kidney transplantation)
- Age
- Sex
- HDL
- Diabetic condition
- Hypertension condition
- Hemoglobin A1c

- In a second model, we also included a weighted genetic risk score (GRS) with variants previously identified to be associated with CHD and not related with classical cardiovascular risk factors (rs10455872, rs12526453, rs1333049, rs17465637, rs501120, rs6725887, rs9818870, rs9982601, rs10507391, rs17222842, and rs9315051).
- GRS was computed for each individual as the sum of the number of risk alleles across 11 variants, after weighting each one by its estimated effect size in the CARDIoGRAM study (Nat Genet. 2011; 43:333)
- We tested for association between incidence of coronary events and clinical data and individual genetic variants and the GRS using Cox proportional hazards models.
- Statistics used to assess the potential value of including the GRS in risk prediction in accordance to AHA criteria were:

  a. Relative risk, hazard ratio for GRS
  b. Discriminative capacity of the model was evaluated using the concordance index (c-statistic)
  c. Accuracy of the model:

  - Goodness-of-fit of the models was evaluated by Hosmer-Lemeshow test - Reclassification improvement was calculated using the net reclassification improvement (NRI) index

Results:

Figure 2 shows how CKD population had a higher cardiovascular and coronary risk than a non-CKD general Spanish population (Rev Esp Cardiol 2003; 56(3):253).

As shown in the following table, among other risk factors, GRS was independently related to coronary events:

Table 2:

| Risk factors for Coronary Events | | | |
|---|---|---|---|
| | No Coronary Events (n = 547) | Coronary Events (n = 73) | p |
| Age (years) | 53 ± 10 | 55 ± 10 | 0.074 |
| Sex (female) | 34.7% | 17.8% | 0.006 |
| Cholesterol (mg/dl) | 209 ± 49 | 206 ± 60 | 0.690 |
| HDL (mg/dl) | 53 ± 17 | 46 ± 16 | 0.001 |
| Systolic BP (mmHg) | 141 ± 20 | 140 ± 20 | 0.955 |
| Diastolic BP (mmHg) | 82 ± 12 | 82 ± 10 | 0.808 |
| Diabetes | 19.7% | 24.7% | 0.409 |
| Hypertension | 84.6% | 83.6% | 0.946 |
| HbA1c (%) | 5.6 ± 0.5 | 5.8 ± 0.6 | 0.149 |
| MDRD (ml/mm/1.73m$^2$) | 19.2 (8.7; 43.6) | 20.5 (10.5; 32.9) | 0.911 |
| Calcium (mg/dl) | 9.3 (8.7; 9.8) | 9.0 (8.3; 9.7) | 0.127 |
| Phosphorus (mg/dl) | 4.2 (3.4; 5.6) | 4.3 (3.4; 5.4) | 0.927 |
| PTH (ng/l) | 156 (72; 307) | 226 (94; 392) | 0.019 |
| Albumin (g/dl) | 4.1 (3.8; 4.4) | 4.1 (3.7; 4.4) | 0.791 |

(continued)

| Risk factors for Coronary Events | | | |
|---|---|---|---|
| | No Coronary Events (n = 547) | Coronary Events (n = 73) | p |
| Standardized GRS | -0.08 (-0.72; 0.68) | 0.30 (-0.21; 0.86) | 0.024 |
| a. Relative risk, hazard ratio for GRS. The hazard ratio for the GRS is 1,29 [1.02;1.64, 95%CI], p=0.035.<br>b. Discriminative capacity of the model was evaluated using the concordance index (c-statistic). As shown in the following table 3, the discrimination of the CHD risk model improved with the GRS<br>c. Accuracy of the model:<br>- Goodness-of-fit of the models was evaluated by Hosmer-Lemeshow test. As shown in table 3, the accuracy was very good with no statistical difference between predicted and observed events.<br>- Reclassification was improved as calculated using the net reclassification improvement (NRI) index, shown in table 3. | | | |

Table 3:

| Predictive Ability for Coronary Events | | |
|---|---|---|
| | CLINICAL MODEL | CLINICAL MODEL + GRS |
| C-index (%) p-value | 67.8 (59.9; 75.6) | 71.7 (64.3; 79.0) 0.015 |
| Hosmer-Lemeshow test p-value | 9.5 (0.092) | 9.6 (0.088) |
| Net Reclassification Improvement (%) | Reference | 22.0 (5.5; 38.6) |

Conclusions:

- A genetic risk score including gene variants associated with CHD but not with classical cardiovascular risk factors independently relates to coronary heart disease in a population of patients with chronic kidney disease
- The inclusion of GRS improves risk prediction of CHD in patients with CKD, specially with KT.
- Genetic screening could help to prevent CHD events in this group of patients.

## Claims

**1.** A method for a cardiovascular risk assessment in a subject comprising the steps of determining in a sample isolated from said subject suffering from chronic kidney disease the presence of polymorphisms at positions 27 within the nucleic acid sequences of SEQ ID NO:1 to 8, wherein the presence at position 27 of a C in SEQ ID NO:1, C in SEQ ID NO:2, T in SEQ ID NO:3, C in SEQ ID NO:4, C in SEQ ID NO:5, A in SEQ ID NO:6, T in SEQ ID NO:7, and G in SEQ ID NO:8, which correspond to db SNP accession number rs17465637, rs6725887, rs9818870, rs12526453, rs1333049, rs501120, rs9982601, rs10455872, respectively, is indicative of a risk of having a cardiovascular event, wherein in addition the presence at position 27 of an A in SEQ ID NO: 9, A in SEQ ID NO:10, and G in SEQ ID NO:11 with db SNP accession no. rs10507391, rs9315051, and rs17222842, respectively, is determined,

- wherein the polymorphism represented by SEQ ID NO:6 (rs501120) can be replaced by the polymorphism represented by SEQ ID NO 49 (rs1746048), and/or wherein the polymorphism represented by SEQ ID NO:1 (rs17465637) can be replaced by the polymorphism represented by SEQ ID NO 48 (rs2133189).

**2.** The method of claim 1, wherein the latter three SEQ (SEQ ID NO 9 - 11) and rs are forming the haplotype B ALOX5AP and are considered as one risk genetic component in addition to the other 8 sequences.

**3.** A method as defined in any of the claims 1 to 2 wherein the cardiovascular event is selected from the group of fatal or non-fatal myocardial infarction, stroke, angina pectoris, transient ischemic attacks, peripheral arterial disease or a combination thereof.

**4.** A method as defined in any of claims 1 to 3 further comprising determining one or more cardiovascular disease or disorder risk factor(s) selected from the group consisting of age, race, sex, body mass index, systolic blood pressure, diastolic blood pressure, smoking status and history, total cholesterol, low density lipoprotein (LDL)- or high density lipoprotein (HDL)-cholesterol level, triglycerides, dyslipemia history, history of heart failure, , previous coronary heart disease, diabetes mellitus, glycemia, glycated hemoglobin, hemoglobin A1c, glomerular filtration, kidney disease status (CKD status 1-4, End Stage Renal Disease (ESRD) in renal replacement therapy or kidney transplantation), hypertension, renal insufficiency and its status (1 to 5), chronic kidney disease and its status (pre-dialysis, dialysis, transplantation, transplantation failure), total time on renal replacement therapy, number of transplants, left ventricular hypertrophy, alcohol consumption, physical activity practice, diet and family history of cardiovascular or coronary disease, creatinine, calcium, phosphorus, parathormone, albumin, and 24 hours proteinuria.

**5.** A method as defined in claims 1 to 4 wherein a plurality of classical risk factors "p" are used, said plurality being selected from the group of:

- Sex, age, Total cholesterol, HDL-cholesterol, blood pressure, diabetes and smoking,
- Age, LDL-cholesterol, HDL-cholesterol, triglycerides, systolic blood pressure, family history of myocardial infarction and diabetes,
- Sex, Log(age/10), total cholesterol/HDL-cholesterol, body mass index, family history of premature CVD, smoking, Townsend score of output area, systolic blood pressure, treatment for hypertension and interaction Systolic Blood Pressure (SBP)*Hypertension (HTN) treatment,
- Kidney disease status (CKD stages 1-4, ESDR in renal replacement therapy or kidney transplantation), age, sex, HDL-cholesterol, diabetic condition, hypertension condition, hemoglobin A1c,
- Age, previous coronary heart disease, smoking, serum creatinine, diabetes mellitus, LDL-cholesterol, total time on renal replacement therapy,
- Age, previous coronary heart disease, smoking, serum creatinine, diabetes mellitus, LDL-cholesterol, total time on renal replacement therapy, number of transplants,
- HDL-cholesterol, diabetes mellitus, hypertension, dyslipemia, hemoglobin A1c, glomerular filtration, calcium, phosphorus, parathormone, albumin.

**6.** A kit comprising reagents for detecting the identity of the nucleotide at position 27 within the nucleic acid sequences of SEQ ID NO: 1 to 11,
which comprises the primer pairs specific for the amplification of a region comprising at least position 27 within the nucleic acid sequences of SEQ ID NO: 1 - 11.

**7.** A kit as defined in claim 6, wherein the selected sequences are SEQ ID NO:1, SEQ ID NO:2, SEQ ID NO:3, SEQ ID NO:4 , SEQ ID NO:5, SEQ ID NO:6, SEQ ID NO:7, SEQ ID NO:8, SEQ ID NO:9, SEQ ID NO:10, and SEQ ID NO:11, with db SNP accession number rs17465637, rs6725887, rs9818870, rs12526453, rs1333049, rs501120, rs9982601, rs10455872, rs10507391, rs9315051, and rs17222842, respectively, ,

- wherein the polymorphism represented by SEQ ID NO:6 (rs501120) can be replaced by the polymorphism represented by SEQ ID NO 49 (rs1746048), and/or wherein the polymorphism represented by SEQ ID NO:1 (rs17465637) can be replaced by the polymorphism represented by SEQ ID NO 48 (rs2133189).

## Patentansprüche

**1.** Verfahren zur Bewertung des Risikos kardiovaskulärer Erkrankungen in einem Subjekt, umfassend die Schritte des Bestimmens des Vorliegens von Polymorphismen an Positionen 27 innerhalb der Nukleinsäuresequenzen von SEQ ID NO: 1 bis 8 in einer aus dem Subjekt, das an chronischer Nierenerkrankung leidet, isolierten Probe, wobei das Vorliegen an Position 27 eines C in SEQ ID NO: 1, C in SEQ ID NO: 2, T in SEQ ID NO: 3, C in SEQ ID NO: 4, C in SEQ ID NO: 5, A in SEQ ID NO: 6, T in SEQ ID NO: 7 und G in SEQ ID NO: 8, die db SNP-Hinterlegungsnummer rs17465637, rs6725887, rs9818870, rs12526453, rs1333049, rs501120, rs9982601 bzw. rs10455872 entsprechen, auf ein Risiko des Eintretens eines kardiovaskulären Ereignisses hinweist, wobei zusätzlich das Vorliegen an Position 27 eines A in SEQ ID NO: 9, A in SEQ ID NO: 10 und G in SEQ ID NO: 11 mit db SNP-Hinterlegungsnr. rs10507391, rs9315051 bzw. rs17222842 bestimmt wird,

- wobei der durch SEQ ID NO: 6 repräsentierte Polymorphismus (rs501120) durch den durch SEQ ID NO: 49 (rs1746048) repräsentierten Polymorphismus ersetzt werden kann, und/oder wobei der durch SEQ ID NO: 1 (rs17465637) repräsentierte Polymorphismus durch den durch SEQ ID NO: 48 (rs2133189) repräsentierten Polymorphismus ersetzt werden kann.

2. Verfahren gemäß Anspruch 1, wobei die letzteren drei SEQ (SEQ ID NO: 9-11) und rs den Haplotyp B ALOX5AP bilden und zusätzlich zu den anderen 8 Sequenzen als eine genetische Risikokomponente betrachtet werden.

3. Verfahren wie in irgendeinem der Ansprüche 1 bis 2 definiert, wobei das kardiovaskuläre Ereignis aus der Gruppe von tödlichem oder nicht-tödlichem Myokardinfarkt, Schlaganfall, Angina pectoris, transitorischen ischämischen Attacken, peripherer arterieller Verschlusskrankheit oder einer Kombination davon ausgewählt ist.

4. Verfahren wie in irgendeinem der Ansprüche 1 bis 3 definiert, weiterhin umfassend das Bestimmen eines oder mehrerer Risikofaktor(s/en) für kardiovaskuläre Erkrankung oder Störung, ausgewählt aus der Gruppe, bestehend aus Alter, Rasse, Geschlecht, Body-Mass-Index, systolischem Blutdruck, diastolischem Blutdruck, Raucherstatus und -vorgeschichte, Gesamtcholesterin, Spiegel von Lipoprotein-Cholesterin niedriger Dichte (LDL) oder Lipoprotein-Cholesterin hoher Dichte (HDL), Triglyceriden, Vorgeschichte von Dyslipidämie, Vorgeschichte von Herzinsuffizienz, früherer koronarer Herzerkrankung, Diabetes mellitus, Glykämie, glycosyliertem Hämoglobin, Hämoglobin-A1c, glomerulärer Filtration, Nierenerkrankungsstatus (CDK-Status 1-4, Nierenerkrankung im Endstadium (End Stage Renal Disease; ESRD) in Nierenersatztherapie oder Nierentransplantation), Hypertonie, Niereninsuffizienz und deren Status (1 bis 5), chronischer Nierenerkrankung und deren Status (Prädialyse, Dialyse, Transplantation, Transplantationsversagen), Gesamtzeit in Nierenersatztherapie, Anzahl von Transplantaten, linksventrikulärer Hypertrophie, Alkoholkonsum, Praxis körperlicher Aktivität, Ernährung und familiärer Vorgeschichte von kardiovaskulärer oder koronarer Erkrankung, Kreatinin, Calcium, Phosphor, Parathormon, Albumin und 24-Stunden-Proteinurie.

5. Verfahren wie in den Ansprüchen 1 bis 4 definiert, wobei eine Mehrzahl klassischer Risikofaktoren "p" verwendet wird, wobei die Mehrzahl aus den folgenden Gruppen ausgewählt ist:

• Alter, Geschlecht, Gesamtcholesterin, HDL-Cholesterin, Blutdruck, Diabetes und Rauchen,
• Alter, LDL-Cholesterin, HDL-Cholesterin, Triglyceride, systolischer Blutdruck, familiäre Vorgeschichte von Myokardinfarkt und Diabetes,
• Geschlecht, Log(Alter/10), Gesamtcholesterin/HDL-Cholesterin, Body-Mass-Index, familiäre Vorgeschichte von vorzeitiger CVD, Rauchen, Townsend-Score des Ausgabebereichs, systolischer Blutdruck, Behandlung für Hypertonie und Behandlung von Interaktion von systolischem Blutdruck (SBP)*Hypertonie (HTN),
• Nierenerkrankungsstatus (CKD-Stadien 1-4, ESRD in Nierenersatztherapie oder Nierentransplantation), Alter, Geschlecht, HDL-Cholesterin, diabetischer Zustand, Hypertonie-Zustand, Hämoglobin-A1c,
• Alter, frühere koronare Herzerkrankung, Rauchen, Serum-Kreatinin, Diabetes mellitus, LDL-Cholesterin, Gesamtzeit in Nierenersatztherapie,
• Alter, frühere koronare Herzerkrankung, Rauchen, Serum-Kreatinin, Diabetes mellitus, LDL-Cholesterin, Gesamtzeit in Nierenersatztherapie, Anzahl von Transplantaten,
• HDL-Cholesterin, Diabetes mellitus, Hypertonie, Dyslipidämie, Hämoglobin-A1c, glomeruläre Filtration, Calcium, Phosphor, Parathormon, Albumin.

6. Kit, umfassend Reagenzien zum Detektieren der Identität des Nukleotids an Position 27 innerhalb der Nukleinsäuresequenzen von SEQ ID NO: 1 bis 11,
welches die Primerpaare umfasst, die für die Amplifikation einer Region, umfassend wenigstens Position 27 innerhalb der Nukleinsäuresequenzen von SEQ ID NO: 1-11, spezifisch sind.

7. Kit wie in Anspruch 6 definiert, wobei die ausgewählten Sequenzen SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 und SEQ ID NO: 11 sind, mit der db SNP-Hinterlegungsnummer rs17465637, rs6725887, rs9818870, rs12526453, rs1333049, rs501120, rs9982601, rs10455872, rs10507391, rs9315051 bzw. rs17222842,

- wobei der durch SEQ ID NO: 6 (rs501120) repräsentierte Polymorphismus durch den durch SEQ ID NO: 49 (rs1746048) repräsentierten Polymorphismus ersetzt werden kann, und/oder wobei der durch SEQ ID NO: 1 (rs17465637) repräsentierte Polymorphismus durch den durch SEQ ID NO: 48 (rs2133189) repräsentierten Polymorphismus ersetzt werden kann.

## Revendications

1. Procédé pour une évaluation du risque cardiovasculaire chez un sujet comprenant les étapes consistant à déterminer dans un échantillon isolé dudit sujet souffrant d’une maladie rénale chronique la présence de polymorphismes aux positions 27 dans les séquences d'acide nucléique SEQ ID NO: 1 à 8, où la présence en position 27 d'un C dans SEQ ID NO: 1, d'un C dans SEQ ID NO:2, d'un T dans SEQ ID NO:3, d'un C dans SEQ ID NO:4, d'un C dans SEQ ID NO:5, d'un A dans SEQ ID NO:6, d'un T dans SEQ ID NO:7 et d'un G dans SEQ ID NO:8, qui correspondent aux numéros d'accès SNP db rs17465637, rs6725887, rs9818870, rs12526453, rs1333049, rs501120, rs9982601, rs10455872, respectivement, est révélateur d'un risque de présenter un événement cardiovasculaire, dans lequel en plus la présence en position 27 d'un A dans SEQ ID NO: 9, d'un A dans SEQ ID NO:10, et d'un G dans SEQ ID NO:11, avec des numéros d'accès SNP db rs10507391, rs9315051 et rs17222842, respectivement, est déterminée,

- dans lequel le polymorphisme représenté par SEQ ID NO:6 (rs501120) peut être remplacé par le polymorphisme représenté par SEQ ID NO: 49 (rs1746048), et/ou dans lequel le polymorphisme représenté par SEQ ID NO:1 (rs17465637) peut être remplacé par le polymorphisme représenté par SEQ ID NO:48 (rs2133189).

**2.** Procédé selon la revendication 1, dans lequel les trois dernières SEQ (SEQ ID NO: 9-11) et rs forment l'haplotype B ALOX5AP et sont considérées comme une composante du risque génétique en plus des 8 autres séquences.

**3.** Procédé tel que défini dans l'une quelconque des revendications 1 à 2 dans lequel l'événement cardiovasculaire est choisi dans le groupe constitué par l'infarctus du myocarde fatal ou non fatal, l'accident vasculaire cérébral, l'angine de poitrine, l'accident ischémique transitoire, l'artériopathie périphérique ou une de leur combinaison.

**4.** Procédé tel que défini dans l'une quelconque des revendications 1 à 3, comprenant en outre une détermination d'un(e) ou plusieurs maladie(s) ou facteur(s) de risque de trouble cardiovasculaire choisis dans le groupe constitué par l'âge, la race, le sexe, l'indice de masse corporelle, la pression artérielle systolique, la pression artérielle diastolique, le statut et les antécédents du tabagisme, le cholestérol total, le niveau des lipoprotéines de basse densité (LDL)- ou des lipoprotéines de haute densité (HDL)- du cholestérol, les triglycérides, les antécédents de dyslipémie, les antécédents d'insuffisance cardiaque, les antécédents de maladie cardiaque coronarienne, le diabète sucré, la glycémie, l'hémoglobine glyquée, l'hémoglobine A1c, la filtration glomérulaire, le statut de maladie rénale (statut CKD 1-4, l'insuffisance rénale au stade ultime (IRSU) en traitement de substitution rénale ou transplantation rénale), l'hypertension, l'insuffisance rénale et son statut (1 à 5), la maladie rénale chronique et son statut (pré-dialyse, dialyse, transplantation, échec de transplantation), la durée totale du traitement de remplacement rénal, le nombre de greffes, l'hypertrophie ventriculaire gauche, la consommation d'alcool, la pratique d'activité physique, le régime alimentaire et les antécédents familiaux de maladie cardiovasculaire ou coronarienne, la créatinine, le calcium, le phosphore, la parathormone, l'albumine, et la protéinurie sur 24 heures.

**5.** Procédé tel que défini dans les revendications 1 à 4, dans lequel une pluralité de facteurs de risque classiques « p » sont utilisés, ladite pluralité étant choisie dans le groupe constitué par:

• le sexe, l'âge, le cholestérol Total, le cholestérol-HDL, la pression artérielle, le diabète et le tabagisme,
• l'âge, le cholestérol-LDL, le cholestérol-HDL, les triglycérides, la tension artérielle systolique, les antécédents familiaux d'infarctus du myocarde et du diabète,
• le sexe, le Log (âge/10), le cholestérol total/cholestérol-HDL, l'indice de masse corporelle, les antécédents familiaux de MCV précoce, le tabagisme, le score de Townsend sur la surface de sortie, la pression artérielle systolique, le traitement de l'hypertension et l'interaction tension artérielle systolique (SBP) * traitement de l'Hypertension (HTN),
• le statut de maladie rénale (stades CKD 1-4, ESDR en traitement rénal substitutif ou transplantation rénale), l'âge, le sexe, le cholestérol HDL, l'état diabétique, l'état d'hypertension, l'hémoglobine A1c,
• l'âge, les antécédents de maladie cardiaque coronarienne, le tabagisme, la créatininémie, le diabète sucré, le cholestérol-LDL, la durée totale d'un traitement de substitution rénale,
• l'âge, les antécédents de maladie cardiaque coronarienne, le tabagisme, la créatininémie, le diabète sucré, le cholestérol-LDL, la durée totale d'un traitement de substitution rénale, le nombre de greffes,
• le cholestérol-HDL, le diabète sucré, l'hypertension, la dyslipémie, l'hémoglobine A1c, la filtration glomérulaire, le calcium, le phosphore, la parathormone, l'albumine.

**6.** Kit comprenant des réactifs pour détecter l'identité du nucléotide en position 27 dans une séquence d'acide nucléique de SEQ ID NO: 1 à 11, qui comprend la paire d'amorces spécifiques pour l'amplification d'une région comprenant au moins la position 27 dans une séquence d'acide nucléique de SEQ ID NO: 1 à 11.

**7.** Kit tel que défini dans la revendication 6, dans lequel les séquences sélectionnées sont SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 et SEQ ID NO: 11, avec les numéros d'accès SNP db rs17465637, rs6725887, rs9818870, rs12526453, rs1333049, rs501120, rs9982601, rs10455872, rs10507391, rs9315051, et rs17222842, respectivement,

- dans lequel le polymorphisme représenté par SEQ ID NO: 6 (rs501120) peut être remplacé par le polymorphisme représenté par SEQ ID NO: 49 (rs1746048), et/ou dans lequel le polymorphisme représenté par SEQ ID NO: 1 (rs17465637) peut être remplacé par le polymorphisme représenté par SEQ ID NO: 48 (rs2133189).

Figure 1

| SEQ ID NO: | Sequence comprising the polymorphism | db SNP accession number | Allele of risk | Chrom | Position in chrom | Strand | Accession number/ Build |
|---|---|---|---|---|---|---|---|
| 1 | AACCATAATAGTTATGCTGAGAAGTT**C**TTTTTTGTCATAGTGCAAGATAACA | rs17465637 | C | 1 | 196042601 | (+) | AC000044/03.03.2008 |
| 2 | GCTATCATTTAAATTTGGTTGAGACA**C**AATATGCTGTTGCACTTTCTATAAA | rs6725887 | C | 2 | 197498571 | (+) | AC000045/03.03.2008 |
| 3 | CTGTGCTGCTTGGTGCCTCTCTGATA**T**GAATACACTGACACGTCAAAGTAAC | rs9818870 | T | 3 | 136547031 | (+) | AC000046/03.03.2008 |
| 4 | ACATCTGCCTCTCTAGACTATAAACT**C**TTTGGGGCTAGGTCTTCTTTGTCTT | rs12526453 | C | 6 | 14155621 | (+) | AC000049/03.03.2008 |
| 5 | TCATACTAACCATATGATCAACAGTT**C**AAAAGCAGCCACTCGCAGAGGTAAG | rs1333049 | C | 9 | 22063860 | (+) | AC000062/03.03.2008 |
| 6 | TTGAAAAAAATTAATTCTCACACTCC**A**AAGTGCATTTAATTTAAGCTACTTT | rs501120 | A | 10 | 44753867 | (-) | AC012277.3 |
| 7 | GGCAAGTACCTGGGCACAGGGCTGCT**T**CATGGCCTTGGACCTGGACAGTGGA | rs9982601 | T | 21 | 35599128 | (+) | AC000064/03.03.2008 |
| 8 | TTCAGACACCTTGTTCTCAGAACCCA**G**TGTGTTTATACAGGTTAGAGGAGAAA | rs10455872 | G | 6 | 161010118 | (+) | |
| 9 | TGTCCAAGCCTCTCTTTGCAATTCTA**A**TTAACCTCAATGTTGCAACCATAGA | rs10507391 | A | 13 | 31312096 | (+) | |
| 10 | CTCATGAACATGACTGTGAACAGGAA**A**ACAGGGAGAGAATGAAGCTGGCCAA | rs9315051 | A | 13 | 31336177 | (+) | |
| 11 | GAGTTTTCCTGGGATGTGGTCCTTTC**G**GTTTTTTAAAAATTATTTTTATTGA | rs17222842 | G | 13 | 31340117 | (+) | |

Figure 2

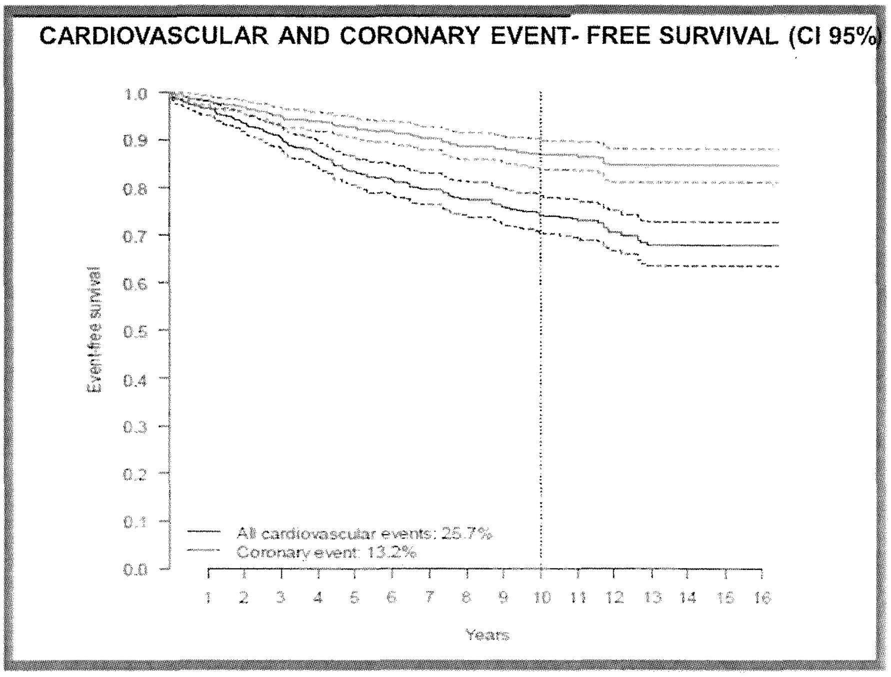

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2554679 A1
- EP 2264183 A1
- WO 2012001613 A1
- WO 2008060618 A2
- US 6159693 A
- US 4683195 A
- US 4683202 A
- US 4800159 A
- US 4883750 A

### Non-patent literature cited in the description


- *Am J Kidney Dis,* 2002, vol. 39 (1), S1-S266
- *J Am Coll Cardiol,* 2007, vol. 50, 217
- *Am J Kidney Dis,* 1998, vol. 32, 992-999
- *erratum,* 2000, vol. 35, 178
- US Renal Data System. USRDS 1998 Annual Data Report. National Institute of Diabetes and Digestive and Kidney Diseases, 1998
- **KAMEL WB et al.** *Am J Cardiol,* 1988, vol. 62, 1109-1112
- **WILSON PWF et al.** *Circulation,* 1988, vol. 97, 1837-1847
- **GRUNDY S.M. et al.** *Circulation,* 1988, vol. 97, 1876-1887
- **GRUNDY S.C. et al.** *Circulation,* 2000, vol. 101, e3-e11
- *Circulation,* 2000, vol. 101, e3-e11
- *J Am Coll Dardiol,* 2007, vol. 50, 217-224
- **PATRICK LINSEL-BITSCHKE et al.** Genetic variations in the arachidonate 5-lipoxygenase-activating protein (ALOX5AP is associated with myocardial infarction in the German population. *CLINICAL SCIENCE,* 01 November 2008, vol. 115 (10), 309
- *FUNDACIO INST DE RECERCA HOSPITAL UNI VALL D HEBRON [ES,* 05 January 2012
- **ANNABEL Z. WANG et al.** Association of SNP rs 17465637 on Chromosome 1q41 and rs599839 on 1p13.3 with Myocardial Infarction in an American Caucasian Population. *ANNALS OF HUMAN GENETICS,* 04 April 2011, vol. 75 (4), 475-482
- Evaluation of pupulation impact of candidate polymorphisms for coronary heart disease in the Framingham Heart Study Offspring Cohort. **YAN YU et al.** BMC PROCEEDINGS. BIOMED CENTRAL LTD, 15 December 2009, vol. 3, S118
- **YUMIKO HIURA et al.** Validation of the Association of Genetic Variants on Chromosome 9p21 and 1p41 With Myocardial Infarction in a Japanese Population. *CIRCULATION JOURNAL,* 01 August 2008, vol. 72 (8), 1213-1217
- Summaries of Key Journal Articles. **EAGLE K A et al.** JOURNAL OF THE AMERICAN COLLEGE OF CARDIOLOGY. ELSEVIER, 02 March 2010, vol. 55, 948-955
- **M RAO et al.** Cytokine gene polymorphisms and progression of renal and cardiovascular diseases. *KIDNEY INTERNATIONAL,* 20 June 2007, vol. 72 (5), 549-556
- Understanding cardiovascular disease through the lense of genome-wide association studies. **ARKING D et al.** TRENDS IN GENETICS. ELSEVIER SCIENCE PUBLISHERS, 01 September 2009, vol. 25, 387-394
- **ROBERT CLARKE et al.** Genetic Variants Associated with Lp(a) Lipoprotein Level and Coronary Disease. *NEW ENGLAND JOURNAL OF MEDICINE,* 24 December 2009, vol. 361 (26), 2518-2525
- *Transplantation,* 2013, vol. 95, 142-147
- *Acad Radiol,* 1997, vol. 4, 49-58
- *Statist Med,* 2008, vol. 27, 157-172
- *Circulation,* 2009, vol. 119, 2408-2416
- *Rev Esp Cardiol,* 2003, vol. 56 (3), 253
- **HILL ; ROBERTSON.** *Theor Appl Genetics,* 1968, vol. 38, 226-231
- *Science,* 1977, vol. 196, 180
- **GRUNSTEIN ; HOGNESS.** *Proc, Natl. Acad. Sci.,* 1975, vol. 72, 3961
- **AUSUBEL et al.** Current Protocols in Molecular Biology. Wiley Interscience, 2001
- **BERGER ; KIMMEL.** Guide to Molecular Cloning Techniques. Academic Press, 1987
- **SAMBROOK et al.** Molecular Cloning: A Laboratory Manual. Cold Spring Harbor Laboratory Press, 1989
- Sequence Analysis Software Package of the Genetics Computer Group. University of Wisconsin Biotechnology Center
- *N Eng J Med,* 2004, vol. 351, 1296
- *Kidney Int,* 2006, vol. 70, 26
- *Nat Genet.,* 2011, vol. 43, 333